# EUROPEAN PATENT APPLICATION

(11) **EP 4 130 244 A1**
(43) Date of publication of application: **08.02.2023**
(21) Application number: 21774433.3
(22) Date of filing: 23.03.2021
(51) Int. Cl.: C12N 5/073, C12N 5/0735, C12N 5/075, C12N 5/076, C12N 5/10, C12N 15/87, C12N 15/877, A61L 27/36, A61L 27/38

(54) **TEMPORARY TREATMENT MEDIUM, TREATMENT KIT, EMBRYO DEVELOPMENTAL ARREST INHIBITOR, METHOD FOR INHIBITING EMBRYO DEVELOPMENTAL ARREST, METHOD FOR PRODUCING DEVELOPMENTAL ENGINEERING PRODUCT, TRANSFER METHOD, THERAPEUTIC METHOD, AND DEVELOPMENTAL ENGINEERING PRODUCT**

(30) Priority: 24.03.2020 JP 2020052748
(71) Applicant: Jichi Medical University, Tokyo 102-0093 (JP)
(72) Inventor: NAGAO, Yasumitsu, Shimotsuke-shi, Tochigi 329-0498 (JP); ENDO, Hitoshi, Shimotsuke-shi, Tochigi 329-0498 (JP); SAKASHITA, Eiji, Shimotsuke-shi, Tochigi 329-0498 (JP); OHMORI, Tsukasa, Shimotsuke-shi, Tochigi 329-0498 (JP); HAYAKAWA, Morisada, Shimotsuke-shi, Tochigi 329-0498 (JP)
(74) Representative: Grünecker Patent- und Rechtsanwälte PartG mbB
(86) International application number: PCT/JP2021/011891
(87) International publication number: WO 2021/193596

(57) **Abstract**

Provided is a temporary treatment medium that suppresses embryogenesis arrest due to manipulation of embryos, and the like. The temporary treatment medium reduces damage from manipulation to in-vitro-cultures containing one or any combination of a pluripotent stem cell, a germ cell, a fertilized egg, and an embryo. Thus, the temporary treatment medium contains an intracellular skeleton regulator and/or an apoptosis inhibitor. The intracellular skeleton regulator and/or apoptosis inhibitor is preferably a Rho kinase inhibitor. Specifically, as the Rho kinase inhibitor, a ROCK inhibitor can be used. This ROCK inhibitor is, for example, Y-27632. The temporary treatment medium is used to treat in-vitro-cultures for a specific period of time before and/or after damaging the manipulation.

## Description

### BACKGROUND

### [Technical field]

The present invention particularly relates to a temporary treatment medium for temporary treatment of an in-vitro-culture, a treatment kit, an embryogenesis arrest inhibitor, an embryogenesis arrest inhibitory method, a developmental engineering product production method, a transplantation method, a therapeutic method, and a developmental engineering product.

### [Background technology]

Typically, developmental engineering and reproductive medicine have been widely used in humans, mice, rats, rabbits, pigs, cows, horses, monkeys, and the like. Gamete freezing and thawing techniques are essential techniques, but it can be inefficient in some species. In case of implementing reproductive technology, there is a technical need that stabilizes gametes such as eggs and can withstand freezing / thawing manipulation, ICSI, nuclear transplantation, chimeric embryo production, gene transfer manipulation, and the like, for example, technology for stabilizing embryos. For example, in recent years, many reports have been made on the production of genetically modified animals by genome editing such as CRISPR-Cas, or the like, as basic research on the production of animal models for diseases disease model animals and their gene therapy.

In order to produce a genetically modified animal, or the like, it is necessary to culture the embryo in vitro and perform embryo manipulation such as modifying the gene.

However, since embryos cultured and manipulated in vitro are damaged, it was often impossible to produce individual embryos depending on the strain or animal species.

Typically, as refer to Non-Patent Document 1, it is described that by introducing recombinant Rho-kinase into cells and enhancing the activity of Rho-kinase, the survival rate of embryos whose cytoskeleton is damaged by freezing is increased. This Rho kinase is a kind of serine / threonine protein-phosphorylating enzyme, and there are two isoforms, ROCK1 and ROCK2, with high homology. By acting on the cytoskeleton, both are primarily associated with important physiological functions, including regulation of cell shape and movement.

In here, according to Non-Patent Document 1, it is described that the survival rate of embryos is significantly reduced by treatment of "Rho-kinase inhibitor" that suppresses the activity of Rho kinase (as refer to the summary, and the like, in the Non-Patent Document 1).

On the other hand, referring to Patent Document 1, a method for forming retinal pigment epithelial cells from pluripotent stem cells such as iPS cells and ES cells by using Y27632, which is a kind of Rho kinase inhibitor, is described.

### [Prior art document]

### [Patent document]

[Patent Document 1] WO 2018/164240
[Patent Document 2] JP2015-70825A

### [Non-patent document]

[Non-Patent Document 1] Gu et al., "Rho/RhoA-associated kinase pathway improves the anti-freezing potentiality of murine hatched and diapaused blastocysts", Sci Rep, US, 2017, 7:6705
[Non-Patent Document 2] Horii, T. et al., "Efficient generation of conditional knockout mice via sequential introduction of lox sites", Sci Rep, (USA), 7: 7891.

### SUMMARY OF THE INVENTION

### [Problems to be Solved by the Invention]

However, the enhancement of Rho-kinase in Non-Patent Document 1 cannot sufficiently suppress embryo damage during the production of genetically modified animals, and it is extremely difficult to produce an individual for a strain sensitive to artificial embryo manipulation with damage.

The present invention has been made in view of such a situation, and an object of the present invention is to solve the above-mentioned problems.

A temporary treatment medium according to the present invention is a temporary treatment medium for reducing manipulation damage to in-vitro-culture including any or any combination of a pluripotent stem cell, a germ cell, a fertilized egg, and an embryo, including: an intracellular skeleton regulator and/or an apoptosis inhibitor.

A treatment kit according to the present invention is a treatment kit including the temporary treatment medium.

An embryogenesis arrest inhibitor according to the present invention is a embryogenesis arrest inhibitor including: an anti-apoptotic agent, which reduces damage caused by manipulation of an in-vitro-culture and suppresses embryogenesis arrest.

An embryogenesis arrest inhibitory method according to the present invention is an embryogenesis arrest inhibitory method that reduces damage caused by manipulation of an in-vitro-culture including any or any combination of a pluripotent stem cell, a germ cell, a fertilized egg, and an embryo, and suppresses embryogenesis arrest, including the step of: treating with a temporary treatment medium including an intracellular skeleton regulator and/or an apoptosis inhibitor for a specific period of time before and/or after a damaging manipulation.

A developmental engineering product production method according to the present invention is a developmental engineering product production method including the step of: preparing a developmental engineering product including one or any combination of an individual, an organ, a tissue, and a cell from the in-vitro-culture treated by the embryogenesis arrest inhibitory method.

A transplantation method according to the present invention is a transplantation method including the step of: transplanting the in-vitro-culture treated by the embryogenesis arrest inhibitory method and/or the developmental engineering product produced by the developmental engineering product production method.

A therapeutic method according to the present invention is a therapeutic method for mammal including the step of: transplanting the in-vitro-culture treated by the embryogenesis arrest inhibitory method and/or the developmental engineering product produced by the method for producing developmental engineering product.

A developmental engineering product according to the present invention is a developmental engineering product, wherein being produced by the method for producing developmental engineering product.

### [Effect of the invention]

According to the present invention, it is possible to provide a temporary treatment medium capable of producing an individual even for a strain sensitive to artificial embryo manipulation or the like accompanied by damage.

### [Simple explanation of drawings]

FIG. 1 is a conceptual diagram showing a flow of an embryogenesis arrest suppressing method according to an embodiment of the present invention;
FIG. 2 is a table summarizing the strain and manipulation results that enable the production of individuals by the embryogenesis arrest inhibitory method and the developmental engineering product production method according to Example 1 of the present invention;
FIG. 3 is a table showing an example of producing a knockout mouse by electroporation by using the C57BL / 6J mouse according to Example 1 of the present invention;
FIG. 4 is a photograph showing electrophoretic patterns to confirm a Flox mouse by a 2-STEP method by using C57BL / 6J according to Example 1 of the present invention;
FIG. 5 is a photograph of an individual of the heterologous mouse Mus spretus (Algerian mouse), which was generated from a frozen egg of the inbred SPR2 (heterologous inbred SPR2 mouse) according to Example 1 of the present invention;
FIG. 6 is a photograph of ES cells established in a heterologous mouse Mus caroli according to Example 1 of the present invention;
FIG. 7 is a photograph of electrophoresis confirming Mus caroli in which the Tyr gene is knocked out according to Example 1 of the present invention;
FIG. 8A is a table showing the state of embryos obtained by genome editing of inbred rats according to Example 1 of the present invention;
FIG. 8B is a table showing the state of an embryos obtained by genome editing of inbred rats according to Example 1 of the present invention;
FIG. 8C is a table showing the state of embryos obtained by genome editing of inbred rats according to Example 1 of the present invention.
FIG. 9A is a photograph of individuals generated by genome editing of a heterologous inbred SPR2 mouse according to Example 1 of the present invention.
FIG. 9B is a photograph of individuals generated by genome editing of a heterologous inbred SPR2 mouse according to Example 1 of the present invention.
FIG. 9C is a photograph of individuals generated by genome editing of a heterologous inbred SPR2 mouse according to Example 1 of the present invention.
FIG. 10 is a photograph of an individual generated by knocking out a homozygous large deletion by electroporation of an inbred rat according to Example 2 of the present invention.

### BEST MODE FOR CARRYING OUT THE INVENTION

### <Embodiment>

In order to produce a genetically modified animal, a pluripotent stem cell, a germ cell, a fertilized egg, an embryo, or the like, (hereinafter referred to as an "in-vitro-culture") is cultured in vitro, and there is a need to perform treatment, operation, manipulation, or the like, by molecular biology, genetic engineering, reproductive engineering, developmental engineering, reproductive medicine, regenerative medicine, and various other technologies (hereinafter, simply referred to as "manipulation"). However, in the manipulation of the in-vitro-culture, the cell is damaged, so that the developmental stage arrest (hereinafter referred to as "embryogenesis arrest") occurs depending on the lineage or animal species. Therefore, in many cases, even an individual could not be produced.

Thus, the present inventors repeated diligent experiments, and they found that it was possible to reduce damage and suppress embryogenesis arrest by treating cells damaged by the manipulation with a temporary treatment medium including an intracellular skeleton regulator and/or an apoptosis inhibitor, and the present invention has been completed.

### [Temporary treatment medium]

The temporary treatment medium according to the present embodiment is a temporary treatment medium for reducing damage caused by manipulation of the in-vitro-culture, and it is characterized by including an intracellular skeleton regulator and/or an apoptosis inhibitor.

Here, the in-vitro-culture according to the present embodiment is used for molecular biology, genetic engineering, reproductive engineering, developmental engineering, reproductive medicine, regenerative medicine, or the like, (hereinafter referred to as "developmental engineering, or the like"), and it includes any one or any combination of an animal pluripotent stem cell, a germ cell, a fertilized egg, and an embryo used in various manipulations and culturing in vitro.

The pluripotent stem cell according to the present embodiment include, for example, a stem cell having pluripotency capable of differentiating into various cells in an organism such as mammal including human and the other vertebrate. Here, the pluripotent stem cell according to the present embodiment is preferable to have properties that are capable of passage and maintain a state in which differentiation does not proceed even after passage, and the karyotype, or the like, is hard to change, or the epigenetic phenotype is hard to change. Further, it is preferable that the pluripotent stem cell according to the present embodiment has a sufficient proliferative ability in vitro or in vivo as related to these features. The specific examples of such pluripotent stem cells according to the present embodiment include an embryonic stem cell (hereinafter referred to as an "ES cell") and an induced pluripotent stem cell (hereinafter referred to as an "iPS cell"), and the other artificially generated or selected pluripotent stem cell, and the like. These pluripotent stem cells according to the present embodiment may be stem cells generated by re-programming the somatic cells with various vectors such as retrovirus, adenovirus, and plasmid containing a specific gene, or with RNAs and low molecular weight compounds.

In addition, as the pluripotent stem cell according to the present embodiment, a naive cell having a higher pluripotency than usual is used, although it does not necessarily have to be a cell having pluripotency close to totipotency is also possible. Further, it is preferable that the pluripotent stem cell according to the present embodiment has a differentiation ability to differentiate into a developmental engineering product as described later, such as being generated as an individual by chimerizing with a germ cell as described below.

Further, the pluripotent stem cell according to the present embodiment is possible to be cultured on a feeder cell layer, on a cell culture plate coated with a basement membrane matrix such as collagen, or the like, and after being maintained, it is possible to obtain this or to obtain a cryopreserved product.

Furthermore, the pluripotent stem cell according to the present embodiment may be a cell generated from cell obtained from a patient with a disease, a cell that serve as a model for the other disease, a cell in which a reporter gene is integrated (reporter cell), a cell capable of conditional knock-out or knock-in, the other gene recombination cell, or the like. The gene recombination includes addition, modification, and deletion of a gene in a chromosome, addition of a gene by a various vector and an artificial chromosome, modification of epigenetic control, addition of artificial genetic substances such as PNA, and other gene recombination.

The germ cell according to the present embodiment may include a primordial germ cell, a spermatocyte, an oocyte, a germ cell before meiosis, the other germ cellderived cell, an egg cell, a sperm, a parthenogenetic cell, a cell capable of forming teratomas different from the pluripotent stem cell as described above, and other cells that may develop ontogenically by some manipulation. As the germ cells according to the present embodiment, those that have been cryopreserved and thawed may be used.

The fertilized egg according to the present embodiment is a fertilized egg in which an animal egg cell and a sperm are fertilized, a partially developed egg, or other developable egg-like cell. The fertilized egg may be fertilized by in vitro fertilization or intracytoplasmic sperm injection, may be a cell acquired at a time when cleavage is just started and omnipotency is present (a cell of a fertilized egg clone), or the like, and the cell that has been cryopreserved and thawed may be used. In the present embodiment, it is possible to use a fertilized egg in a form generally used by those skilled in the art.

The embryo according to the present embodiment is a cell mass at a stage where the number of cells is increased from a fertilized egg by cleavage and reaches a certain number. The embryo according to the present embodiment is also possible to be applied to a split embryo, for example, an embryo such as an embryo obtained by splitting a two-cell stage embryo into two. Alternatively, the embryo according to the present embodiment may be a split embryo, for example, an embryo such as an embryo obtained by dividing a two-cell stage embryo into two, or an embryo developed into a morula or a gastrula. Alternatively, the embryo according to the present embodiment may be a blastocyst, or the like.

Here, the embryo according to the present embodiment may be an embryo derived from a cell having omnipotency, which is derived by using a primordial reproductive cell, or the like, prepared from the pluripotent stem cell. In addition, the mammalian embryo of the subject according to the present embodiment may be a chimera in which cells derived from different kind of animals are mixed. In this case, a chimeric embryo, which may be combined with cells of an animal other than a mammal is possible.

Further, as the target of the embryo treatment according to the present embodiment, it can be applied not only to an embryo but also to an embryoid body that does not necessarily grow into an individual or a fetus but differentiates only into a tissue or an organ of each lineage (series). In such case, an embryo in which a part of the embryo is removed for analysis, that is, a biopsy embryo is also applicable.

Further, as the embryo according to the present embodiment, an embryo that has been cryopreserved and thawed may be used. This cryopreservation may be applied for a blastocyst as common to those skilled in the art.

That is, as the in-vitro-culture according to the present embodiment, one that has been cryopreserved and thawed may be used.

The method for obtaining the embryo according to the present embodiment is not particularly limited. For example, the embryo according to the present embodiment can be obtained by in vivo fertilization, in vitro fertilization, and nuclear transplantation.

In addition, the mammalian embryo according to the present embodiment is a mammalian embryo, which genetic information has been processed by a method such as transgenic in which a gene has been applied by a gene recombination method, gene knockout, conditional knockout, or the like, by using various vectors, or the like. The processing of genetic information may be a gene transfer or removal from the genome by genome editing, or the like, an extrachromosomal gene transfer such as plasmids and artificial chromosomes, an epigenetic control such as control of methylation at specific sites on chromosomes or modification of histones, an addition of PNA or an artificial base, and other various genetic information processing techniques can be used.

Here, the in-vitro-culture according to the present embodiment may be selected in the form of a colony, or the like, by various markers, visual inspection, or the like. Further, the in-vitro-culture according to the present embodiment may be a mixed cell population, a tissue, an organ, or the like (hereinafter, referred to as "tissue or the like"). These in-vitro-cultures may include a mixture of those in various differentiation and developmental states. That is, each cell for the in-vitro-culture may be underdeveloped or immature at the stage of development.

The animal for the in-vitro-culture according to the present embodiment is not particularly limited, and it includes a wide range of vertebrates and invertebrates. The vertebrates include fish, amphibians, reptiles, birds, and mammals.

The mammals to be treated with the embryos according to the present embodiment is originated from, for example, Primates, Rodentia, Lagomorpha, Cetartiodactyla, Perissodactyla, or Carnivora, and, for example, they can be treated with the temporary treatment medium according to the present embodiment, which is prepared for different embryos for each order and species. In addition, all the embryos of Eutheria placental mammals, including rare mammals other than the orders, can be applied as the target of embryo treatment according to the present embodiment.

As explaining the animal that to which the in-vitro-culture belongs according to the present embodiment from a viewpoint different from the above-mentioned order class, for example, human (Homo sapiens), an experimental animal, a domestic animal, a companion animal, and the like, can be enumerated. Among these, as experimental animals, examples of animals of the order Rodentia include a mouse (Mus musculus), a rat (Rattus novegicus), a hamster (Mesocricetus auratus), and a guinea pig (Cavia porcellus), and the like. Examples of animals of the order Leporinae include a rabbit (Leporinae Trouessart), and the like. As domestic animals, examples of animals of the order Artiodactyla include a pig (Sus scrofa domestics), a cattle (Bos taurus), a sheep (Ovis aries), and the like. Examples of animals of the order Perissodactyla include a horse (Equus cavallus), and the like. As companion animals, examples of animals of the order Carnivora include a cat (Felis silvestris catus), a dog (Canis lupus familiaris), a ferret (Mustella putorius), and the like. Examples of non-human animals of the order Primates include a Gorilla and a chimpanzee (Pan troglodytes), which are apes, and a rhesus monkey (Macaca mulatta), the other simians (Simiiformes), and the other primates, and the like. In addition to these, heterogeneous species of animals different from the above examples, for example, as the order Rodentia, Ryukyu mouse (Mus caroli), Algerian mouse (Mus spretus), which are described in the examples later, and their subspecies, and the like are also enumerated. Among these, the subspecies according to the present embodiment may be a subspecies in classification, a subspecies in which the sequence of mitochondrial DNA, or the like, is at least partially different from the well-known species. Further, as a so-called "wild species", a species or a subspecies having a variation in genomic information may be set. In addition to these, the heterogeneous species of the different order described above may also be included. In the present embodiment, these animals of the heterogeneous species are referred to in association with animals having similar appearances and properties, such as "heterogeneous mouse", "heterogeneous rat", and "heterogeneous cat" for convenience. Further, the above-mentioned classification of experimental animals, domestic animals, companion animals, and the like, is for convenience, and it is also used for different purposes, such as breeding purposes, medical purposes, and the like. Further, the animal according to the present embodiment may be an animal that has already been genetically modified, an animal that has undergone genetic modification by mating, a systematized animal, or another genetically modified animal.

Further, the animal according to the present embodiment, also as invertebrates, includes a wide range of animals such as chordates, molluscs, annelids, and arthropods, and the like, which develops with cleavage.

The animal to which the in-vitro-culture according to the present embodiment belongs may be derived from a mammal of a strain that is particularly sensitive to the manipulation according to the present embodiment and is prone to embryogenesis arrest. Furthermore, as shown in the example as described later, such mammal may be a hybrid that is a hybrid strain with clear genetic background or an inbred strain, which is a strain of animal obtained by continuing inbreeding between brothers and sisters for 20 or more generations, or the like. Further, it may be an animal of mutant (genetic mutating) strain such as a nude mouse or the like, an animal of specified disease model, an animal of heterogeneous species derived from originating species, an animal of hybrid between species, or the like. Furthermore, it also includes an animal that been fixed as species by breeding artificially and selectively, subspecies, or the like.

Here, the manipulation on the in-vitro-culture according to the present embodiment (artificial embryo manipulation, or the like, hereinafter simply referred to as "manipulation") includes a treatment involving damage to the in-vitro-culture necessary for developmental engineering, and the like. The manipulation includes not only the manipulation on the in-vitro-culture itself, but also the manipulation on the mother, germ cells, or the like, associated with the acquisition of the in-vitro-culture. In addition, the damage of the in-vitro-culture includes the damage caused by various manipulations such as experiments and treatments after the acquisition of the in-vitro-culture. Specifically, the damage includes damage, alteration, or the like, to various structures necessary for survival and normal differentiation of cells, which are intracellular organelles including nuclei, DNA having genes, intracellular skeletons, various other intracellular structures, cell membranes, and extracellular matrix that are extracellular structures, or the like. In particular, in the present embodiment, the manipulation may be such that causes a nick or a double strand break (DSB) in the DNA in the nucleus due to physical or organic stress applied to a cell, reactive oxygen species due to ionization, destruction of intracellular organelles, or the like, damage by chemical substances, or the like.

Further, these damages may be at least partially recoverable by cell metabolism. Conversely, if a cell does not recover from such damage, it causes cell cycle arrest, differentiation arrest or alteration, apoptosis, necrosis, or other unusual conditions.

Specifically, according to the present embodiment, the manipulation on the in-vitro-culture as a treatment involving damage to the embryo (cell) includes, for example, ovarian hyperstimulation to the mother for easy supply of embryos, freezing or thawing of embryos accompanying the transfer of frozen embryos, dissociation of cells, nuclear transplantation (NT), intracytoplasmic sperm injection (cytoplasmic sperm injection method, in vitro fertilization, intracytoplasmic sperm injection, ICSI), microinjection (MI), electroporation (electroporation method, EP), and the like. Alternatively, the treatment involving damage to the nucleus and DNA in the nucleus includes exposure to a large amount or high molecular weight substance, (long sequence) DNA or RNA, treatment involving multiple DSBs as described later, and the like. Further, manipulations such as cell fusion, and the like, are also included in the manipulations according to the present embodiment. The cell fusion, and the like, also include the production of polyploid cells such as aneuploids, tetraploids, and the like.

In addition to these, the manipulation on the in-vitro-culture according to the present embodiment includes various treatments such as osmotic pressure change, perforation with other chemical substances, perforation with forceps and the like, in vitro fertilization (IVF), or the like. As described above, these treatments include those performed for transferring or introducing nuclei, chromosomes, DNA, RNA, and the like, into in-vitro-cultures for the purpose of developmental engineering, or the like. This introduction may be performed by using various media. As the medium, for example, various methods for transferring or introducing such as a plasmid, a viral vector, a Drug Delivery System (DDS) such as a liposome and the like, or other polymer into the cell may be used. Among these, the viral vector may be constructed by using a virus common to those skilled in the art, such as adenovirus, adeno-associated virus, retrovirus, and the like. Further, when using these media, the above-mentioned manipulation may be performed. Alternatively, the above-mentioned manipulation can be performed in order to produce the above-mentioned pluripotent stem cells, and the like. Further, various processes related to in vitro maturation (IVM), embryo transfer (Blastocyst Transfer, BT), and the like, may also be included in the manipulation according to the present embodiment. The embryo transfer also includes surgical transfer to the fallopian tube, transplantation to the non-surgical uterus, and the like, which are common to those skilled in the art.

More specifically, the manipulation on the in-vitro-culture according to the present embodiment also includes various methods for performing a treatment that causes great damage to the in-vitro-culture, such as a treatment involving a plurality of DSBs. The examples of the treatment involving a plurality of DSBs include the production of an animal having gene-mutation on a plurality of sites. About the animal having gene-mutation on a plurality of sites, the method for producing animals also includes, for example, a gene knockout, a knock-in, a conditional knockout, or the like. More specifically, a 2STEP method for producing a conditional knockout animal is also included. The 2STEP method is a method in which electroporation is continuously performed and a Flox sequence is inserted into two sites by genome editing (as refer to, for example, Non-Patent Document 2). This makes it possible to produce an animal having a genetic locus in which the target gene region is sandwiched between the loxP, which is Cre recombinase target sequence (Flox animal).

The intracellular skeleton regulator according to the present embodiment is a substance that regulates the polymerization, depolymerization, assembly, dissociation, action, or the like, for proteins related to the intracellular skeleton. The intracellular skeleton according to the present embodiment includes a polymer, aggregate, or the like, of structural proteins such as cytoskeleton, nuclear skeleton, membrane structure, and other structural proteins such as actin filaments, microtubules, and intermediate filaments configuring the intracellular skeletal structure, or the like. Specifically, the intracellular skeleton regulator according to the present embodiment may be, for example, a substance involved in the polymerization or depolymerization of actin and myosin inside and outside the nucleus related to DSB repair. Here, it is known that the activity of DSB repair differs depending on the cell cycle, and the major cell cycle in the developmental stage from the fertilized egg to the early embryo differs from that of somatic cells. Therefore, as the intracellular skeleton regulator according to the present embodiment, it is possible to use a substance that regulates the cell cycle according to the period and the ratio of these cell cycles. That is, by adjusting the polymerization, depolymerization, aggregation, dissociation, or the like, for the intracellular skeleton with the intracellular skeleton regulator, it is possible to promote the DSB repair of the DNA in the nucleus damaged by the manipulation as described later. This makes it possible to prevent the arrest of development due to damage.

In addition, the apoptosis inhibitor according to the present embodiment is a substance that delays and/or suppresses apoptosis of cells in the developmental stage or the maintenance stage. This includes substances that have the effect and action of delaying and/or suppressing apoptosis associated with DSB repair and cell cycle regulation in the above-mentioned intracellular skeleton regulator. That is, the intracellular skeleton regulator and the apoptosis inhibitor may be the same substance.

Here, the intracellular skeleton regulator and/or apoptosis inhibitor according to the present embodiment is, for example, a protein, a nucleic acid, a low molecular weight compound, various other organic compounds, and the like, and is not particularly limited.

In the present embodiment, it is preferable to use, for example, an inhibitor of Rho kinase (Rock1 or Rock2) as the intracellular skeleton regulator and/or apoptosis inhibitor.

As this Rho kinase inhibitor, for example, it is preferable to use a ROCK inhibitor.

The ROCK inhibitor is, for example, Y-27632 (trans-4-[(1R)-1-Aminoethyl]-N-4-pyrid inylcyclohexanecarboxamide), Fasudil (1-(5-isoquinolinesulfonyl)homopiperazine), or H-1152((S)-(+)-4-Glycyl-2-methyl-1-[(4-methyl-5-isoquinolinyl)sulfonyl]-h exahydro-1H-1,4-diazepine), and the like, can be used. However, it is not limited to these. The other ROCK inhibitors can also be preferably used.

The concentration of the ROCK inhibitor included in the temporary treatment medium according to the present embodiment can be appropriately set by those skilled in the art according to the type, state, density, type and content of manipulation, other condition and the like of the in-vitro-culture. The concentration of the ROCK inhibitor can be used at a low concentration, for example, about 1/2 to 1/100 of the "optimal" concentration of 20 µM (as refer to p. 6, paragraph 4, or the like, in Non-Patent Document 1). That is, the ROCK inhibitor does not necessarily have to be at a concentration that completely suppresses the activity of Rho kinase.

Specifically, the preferable concentration of the ROCK inhibitor contained in the temporary treatment medium according to the present embodiment is, for example, 0.1 µM to 20 µM, preferably 5 µM to 15 µM, and more preferably 8 µM to 12 µM for Y-27632. In the examples as described later, an example in which Y-27632 is temporarily treated with a temporary treatment medium contained at a concentration of 10 µM is described.

In addition to this, in the present embodiment, it is also possible to use an intracellular skeleton regulator having no or little anti-apoptosis effect in normal cells.

In the present embodiment, as such an intracellular skeleton regulator, for example, cytochalasin B (Cytochalasin B, CAS number: 14930-96-2, 2H-Oxacyclotetradecino[2,3-d] isoindole-2,18(5H)-dione, 6,7,8,9,10,12a, 13,14,15,15a,16,17-dodecahydro-5,13-dihydroxy-9, 15-dimethyl-14-methylene-16-(phenylmethyl)-, (3E,5R,9R,11E,12aS,13S,15S,15aS,16S,18aS)-) can also be used.

When the cytochalasin B is used, the same effect can be obtained even at a dose such as 1/20 to 1/5 of Y-27632. Specifically, the cytochalasin B is preferably used as 0.01 µM to 15 µM, preferably 1 µM to 12 µM, and more preferably 3 µM to 8 µM. That is, better results can be obtained when the amount of cytochalasin B is several µM less than that of Y-27632.

In the case of other intracellular skeleton regulators and/or apoptosis inhibitors, it can be set from the above concentration range. In addition, the concentration of the intracellular skeleton regulator and/or the apoptosis inhibitor according to the present embodiment may be constant in each period as described later, may be changed in each period, and may be changed stepwise in each period.

In addition, the intracellular skeleton regulator and/or apoptosis inhibitor according to the present embodiment is an example of the embryogenesis arrest inhibitor according to the present embodiment. As mentioned above, these embryogenesis arrest inhibitors reduce the damage caused by manipulation of in-vitro-cultures, which includes one or any combination of a pluripotent stem cell, a germ cell, a fertilized egg, and an embryo, and suppress embryogenesis arrest. In addition, the embryogenesis arrest inhibitor according to the present embodiment is used for the temporary treatment medium according to the present embodiment.

In addition, the temporary treatment medium according to the present embodiment contains components according to the type, state, density, type and content of manipulation, other condition, or the like of the in-vitro-culture.

The component may include, for example, a component for configuring a medium necessary for culturing an in-vitro-culture and water. For example, the temporary treatment medium according to the present embodiment may be used by adding a pH buffer compound, amino acids, vitamins, antioxidants, antibiotics, collagen precursors, trace metal ions and complexes, various salts, and the like.

More specifically, the temporary treatment medium according to the present embodiment may contain, for example, components of a medium commonly used by those skilled in the art for culturing in-vitro-cultures. As this medium, for example, a medium such as general DMEM (Dulbecco's Modified Eagle Medium), or the like, can be used. Alternatively, it is possible to use a medium containing a specific component specialized for pluripotent stem cells, germ cells, fertilized eggs, embryos, and the like. In addition, the medium may contain serum and various serum substitutes. The medium containing the various serum substitutes may be used in a culture system that is xenogeneic component free (Xeno-Free, XF, or Animal Component-Free, ACF).

Further, the medium may also contain various RNAs, peptides, proteins and the like for promoting differentiation and growth. These include various differentiationinducing factors, growth factors, and the like. Further, depending on the type of manipulation, if necessary, a low-molecular-weight compound for inducing differentiation such as retinoic acid, or the like, may be included.

Alternatively, the temporary treatment medium according to the present embodiment may contain only a component that prevents cells from dying in a short period of time, such as PBS.

### [Treating kit]

The treatment kit according to the present embodiment has a feature including the above-mentioned temporary treatment medium. In addition to this, the treating kit according to the present embodiment may contain a solution (solution for use) for manipulation according to various manipulations, a normal medium used for normal culture, and a medium for manipulation according to the type of manipulation (Hereinafter referred to as "manipulation solution".), and other reagents necessary for manipulation. Such reagents include, for example, the probes and primers according to the present embodiment, various enzymes, buffer solutions, washing solutions, lysates, reagents for test, and the like.

In addition, an in-vitro-culture, a container, other materials, equipment, tools, or the like, necessary for the manipulation according to the present embodiment may be added and provided as a treating kit according to the present embodiment. In addition, the treatment kit according to the present embodiment may include reagents, food, cages, drinking water, and the like for maintaining the developmental engineering products as described later.

In addition, it is also possible to be configured to provide a treatment kit containing a carrier and other reagents necessary for the treatment as described later.

Furthermore, it is also possible to provide a treatment kit, which attaches the intracellular skeleton regulator and/or the apoptosis inhibitor that is added to a normal medium to complete the temporary treatment medium with a manual that describes the concentration and treatment method, and the like.

### [Method for suppressing embryogenesis arrest]

The method for suppressing embryogenesis arrest according to the present embodiment is a method for suppressing embryogenesis arrest by reducing damage caused by manipulation of the in-vitro-culture. Specifically, the method for suppressing embryogenesis arrest according to the present embodiment has a feature for treating with a temporary treatment medium containing the intracellular skeleton regulator and/or the apoptosis inhibitor for a specific period before and/or after the manipulation.

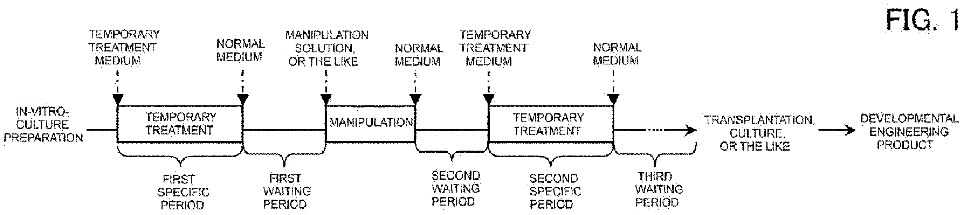

Specifically, with reference to FIG. 1, the major processing flow of the embryogenesis arrest inhibitory method according to the present embodiment is described.

In the embryogenesis arrest inhibitory method according to the present embodiment, appropriately setting the treatment period and term is preferable. Specifically, after preparing the in-vitro-culture, for the first specific period, temporary treatment is performed with the temporary treatment medium according to the present embodiment. Then, for the first waiting period, the medium is replaced to the normal medium or the manipulation solution, and, after waiting, the manipulation is performed. After that, for the second specific period, the medium is replaced with the normal medium according to the present embodiment and waits. Then, the temporary treatment is performed again in place of the temporary treatment medium according to the present embodiment. Further, in some cases, after waiting for the third waiting period, the subsequent processing is performed.

More specifically, first, in the embryogenesis arrest suppressing method according to the present embodiment, an in-vitro-culture to be treated is prepared. This preparation includes various preparatory treatments such as collection of in-vitro-culture, thawing of cryopreserved ones, pick-up of colonies, dissociation of cell masses, and preparation of a drop in which the culture solution is coated with mineral oil, or the like.

Then, treatment with the temporary treatment medium according to the present embodiment is performed only for the first specific period before the manipulation on the prepared in-vitro-culture. This first specific period can be appropriately set according to the type of in-vitro-culture and the type of manipulation to be performed later. The first specific period is, for example, preferably about 1 minute to 2 hours, more preferably about 15 minutes to 1 hour, and even more preferably about 30 minutes to 1 hour. By performing this temporary treatment for the first specific period, the effect of suppressing embryogenesis arrest of the in-vitro-culture can be enhanced. In addition, depending on the species and strain, the temporary treatment for the first specific period may not be performed.

Then, the in-vitro-culture treated with this temporary treatment medium is collected by a centrifuge, or the like, washed with a minimum medium containing less serum or a washing solution such as PBS (Phosphate Buffered Saline), or the like, recovered again, and then transferred to the normal medium. This normal medium is a medium usually used before the manipulation, and it is appropriately set depending on the type of manipulation such as DMEM medium, serum-free medium, and PBS itself. Then, the in-vitro-culture transferred to the normal medium is waited for the first waiting period until the manipulation. The first waiting period is preferably, for example, about 1 minute to 2 hours, and more preferably about 15 minutes to 1 hour. If it is longer than this range, the effect of suppressing embryogenesis arrest of the in-vitro-culture may not be sufficiently obtained.

In addition, the first waiting period may not be necessary. That is, the temporary treatment medium may be washed, replaced with manipulation solution (medium use for manipulation, or the like) or the normal medium, and the in-vitro-culture may be added to perform the manipulation as it is.

Next, the above-mentioned manipulation on the in-vitro-culture is performed. This manipulation may cause damage to the in-vitro-culture. At this time, depending on the type of manipulation, the manipulation is performed after the in-vitro-culture is added to the manipulation solution. In addition, depending on the type of manipulation, it is also possible to perform the manipulation with the normal medium or the temporary treatment medium as it is.

The manipulated in-vitro-culture is then harvested, transferred to normal medium, and waited for the second waiting period. This second waiting period can be appropriately set depending on the type and content of the manipulation. Specifically, the second waiting period is preferably, for example, about 1 minute to 2 hours, and more preferably about 15 minutes to 1 hour. Alternatively, the following temporary treatment can be performed immediately without passing through this second waiting period. That is, the second waiting period may not be necessary.

Here, the treatment with the temporary treatment medium according to the present embodiment is performed only for the second specific period before the manipulation on the in-vitro-culture. This second specific period can be adjusted by a person skilled in the art to an optimum value depending on the animal species, strain, type of manipulation, and the like. Specifically, when using an in-vitro-culture such as a non-inbred strain that is unlikely to cause embryogenesis arrest by performing manipulation, that is, which is less sensitive to manipulation, the second specific period may possible be longer than the first specific period. For example, unfrozen and inbred mice and rats can be temporarily treated with the temporary treatment medium according to the present embodiment for a long time such as 1 to 12 hours as the second specific period. In this case, the effect of suppressing embryogenesis arrest is higher when the time is specifically longer than 1 hour. Alternatively, the pig can be temporarily treated with the temporary treatment medium according to the present embodiment for a long period of 1 hour to 3 days as the second specific period.

Conversely, when by using inbred strains or in-vitro-cultures that are sensitive to manipulations such as inbred strains, the second specific period should be about the same as or slightly longer than the first specific period, and, after that, by using the normal medium, it is better to wait for the third waiting period as described later. Similarly, when frozen eggs and embryos are used, they are less resistant to manipulation damage than unfrozen in-vitro-cultures, and embryogenesis arrest is more likely to occur due to damage to DNA, that is, they are sensitive to manipulation, and that means, it is preferable to shorten the specific period.

With such a configuration, the effect of suppressing embryogenesis arrest can be further enhanced.

The manipulated in-vitro-culture is then collected and transferred to the normal medium. Then, depending on the case, a manipulation such as transplantation to the uterus may be performed after waiting for the third waiting period.

Further, after that, it waits until the developmental stage and is acquired as a developmental engineering product as described later.

During the specific periods of treatment with the temporary treatment medium, the intracellular skeleton regulator and/or the apoptosis inhibitor is permeated into the cell, by enhancing the activity of DSB repair in the nuclear skeleton, by adjusting the cell cycle to the cell cycle in which the activity of DSB repair is high, and by adjusting the polymerization and depolymerization of the intracellular skeletal structure that accompanies the progress of the cell cycle, it is presumed that resistance to damage caused by manipulation is increased because of suppressing pathways such as apoptosis and cell cycle arrest that are normally triggered by damage. Further, it is presumed that embryogenesis arrest is suppressed by being transferred to the normal medium, by suppressing the adverse effects of the intracellular skeleton regulator and/or the apoptosis inhibitor, by restoring the cell cycle to normal, by returning the polymerization and depolymerization of the endoskeleton to normal levels, and by repairing the cell itself.

The optimum value for which of the above-mentioned periods are used and the length of each period can be adjusted by those skilled in the art depending on the animal species, strain, type of manipulation, and the like.

The following is a summary of specific examples of actual processing time.

**[Table 1]**

| ANIMAL SPECIES | STRAIN | FIRST SPECIFIED PERIOD | FIRST WAITING PERIOD | SECOND WAITING PERIOD | SECOND SPECIFIED PERIOD | THIRD WAITING PERIOD |
|---|---|---|---|---|---|---|
| TREATMENT | | ADDED MEDIUM | NORMAL MEDIUM | NORMAL MEDIUM | ADDED MEDIUM | |
| MOUSE RAT | UNFROZEN INBRED STRAIN | 30 MINUTES TO 1 HOUR | 0 TO 1 HOUR | 0 TO 1 HOUR | 1 TO 12 HOURS | |
| | FROZEN INBRED STRAIN UNFROZEN SPECIAL STRAIN | 30 MINUTES TO 1 HOUR | 0 TO 1 HOUR | 0 TO 1 HOUR | 30 MINUTES TO 1 HOUR | 1 TO 12 HOURS |
| | FROZEN AND UNFROZEN WILD MOUSE INBRED STRAIN | 30 MINUTES TO 1 HOUR | 0 TO 1 HOUR | 0 TO 1 HOUR | 30 MINUTES TO 1 HOUR | 1 TO 12 HOURS |
| PIG | IVM, IVF EMBRYO | 30 MINUTES TO 1 HOUR | 0 | 0 | 1 HOUR TO 3 DAYS | |

As shown in this table, in a mouse or a rat, in the case of unfrozen inbred strains by using unfrozen eggs or embryos, it is preferable that the first specific period is 30 minutes to 1 hour, the first waiting period is 0 to 1 hour, the second waiting period is 0 to 1 hour, and the second specific period is 1 to 12 hours. Alternatively, in the case of a frozen inbred strain by using frozen eggs or embryos, it is preferable that the first specific period is 30 minutes to 1 hour, the first waiting period is 0 to 1 hour, the second waiting period is 0 to 1 hour, and the second specific period is 30 minutes to 1 hour, and the third waiting period is preferably 1 to 12 hours. Alternatively, in the case of inbred strains such as a frozen or unfrozen wild mouse or rat, a heterologous mouse or rat, it is preferable that the first specific period is 30 minutes to 1 hour, the first waiting period is 0 to 1 hour, and the second waiting period is 0 to 1 hour, the second specific period is 30 minutes to 1 hour, and the third waiting period is 1 to 12 hours. In pigs, in the case of an IVM or IVF embryo, it is preferable that the first specific period is 30 minutes to 1 hour, the first waiting period is 0 hours, the second specific period is 0 hours, and the second specific period is 1 hour to 3 days.

As described above, the treatment with the temporary treatment medium may be performed only before the manipulation or only after the manipulation. By treating both the temporary treatment medium before and after the manipulation, it is possible to enhance the effect of suppressing embryogenesis arrest depending on the animal species, strain, and type of manipulation. In addition, when the treatment with the temporary treatment medium is performed before or after the manipulation, the first specific period, the first waiting period, the second waiting period, and the second specific period may be different, and each the concentration of intracellular skeleton regulator and/or apoptosis inhibitor contained in the temporary treatment medium may also be different at that period, respectively. These can be optimized and adjusted by those skilled in the art.

Further, the treatment with the temporary treatment medium may be performed a plurality of times before and/or after the manipulation. In this case, it is preferable to provide a waiting period after changing to the normal medium after the treatment.

[Developmental engineering product production method and developmental engineering product]

The developmental engineering product production method according to the present embodiment has a feature that the developmental engineering product is produced from the in-vitro-culture treated by the above-mentioned method for suppressing embryogenesis.

The developmental engineering product according to the present embodiment has a feature being produced by the above-mentioned developmental engineering product production method.

Here, the developmental engineering products according to the present embodiment include one or any combination of an individual, an organ, a tissue, and a cell. Among these, the individual includes a chimeric individual, a model organism, other individual necessary for an experiment, reproductive engineering, and medical care. The organ and the tissue do not necessarily have to be mature to the level of internal organ, as long as specific differentiated cells provide a specific structure as a cell mass. Further, the cell includes dissociated cell mass that do not have a particular structure.

The developmental engineering products produced by the present embodiment can be obtained from animals, strains, or the like, which cannot be achieved by conventional methods, and can further be distinguished by those skilled in the art because mutations due to damage are less. However, in the field to which a person skilled in the art according to the present embodiment belongs, there is a special circumstance that it is very difficult for a person skilled in the art to directly specify it due to its structure or characteristics.

### [Drug discovery support method]

The drug discovery support method according to the present embodiment has a feature that the developmental engineering product produced by the developmental engineering product production method according to the present embodiment is evaluated.

In addition, the drug discovery support method according to the present embodiment can administer a drug related to toxicity and/or disease for drug discovery to a developmental engineering product and evaluate the state of the developmental engineering product.

As the drug for toxicity and/or disease for drug discovery according to the present embodiment, a candidate drug for drug screening of which toxicity needs to be investigated, a candidate drug for treating a disease, and the like, can be used. Examples of the candidate drug according to the present embodiment include a low molecular weight compound, a peptide, a protein, an extract, a supernatant, and a fermented product of a cell, other synthetic compound, a natural compound, and the like. The purity, degree of purification, and the like of these candidate drugs may be arbitrary. Further, the diseases targeted by the drug screening according to the present embodiment include arbitrary diseases.

Among these, toxicity can be evaluated by performing expression analysis, morphological analysis, or the like, of marker genes of developmental engineering products. In addition, screening may be performed according to the protocol of the clinical trial or using any method for those skilled in the art.

In these analyses, it can be estimated that the candidate drug is less toxic when the normal function is maintained in the cells to which the candidate drug is administered.

### [Therapeutic method, transplantation method, and medicine]

The therapeutic method according to the present embodiment is a therapeutic method for mammals and has a feature of transplanting an in-vitro-culture treated by the above-mentioned embryogenesis arrest inhibitory method and/or a developmental engineering product prepared by the above-mentioned developmental engineering product production method.

The therapeutic method according to the present embodiment can be used for reproductive medicine by applying the embryogenesis arrest inhibitory method according to the present embodiment to, for example, a frozen fertilized egg or a blastocyst. This makes it applicable to mothers whose embryos are difficult to settle due to, for example, genetic background, old age, various diseases, and the like. At this time, by transplanting the developmental engineering product according to the present embodiment into the uterus, the transplantation method according to the present embodiment also functions as a therapeutic method.

Alternatively, as the treatment method according to the present embodiment, the developmental engineering product itself, or a processed or extracted product produced by the above-mentioned developmental engineering product production method is obtained as a medicine (medical composition) according to the present embodiment, and it may be used therapeutically. That is, in the therapeutic method according to the present embodiment, it can be used as regenerative medicine for treating diseases of animals including humans.

In the treatment method according to the present embodiment, firstly, the developmental engineering product according to the present embodiment is obtained from a pluripotent stem cell prepared or generated from a patient, or from a library of pluripotent stem cells with similar types such as HLA, and the like. These cells may be produced by the above-mentioned manipulations, may be operated on those produced, and may be treated with the above-mentioned temporary treatment medium before and/or after these manipulations. The obtained pluripotent stem cell, and the like, is induced to differentiate and then cultured for a specific period, and it is obtained as developmental engineering products according to the present embodiment at any stage of a cell, a cell mass, a tissue, an organ, and the like. The acquired developmental engineering product may be dissociated, or the like, and processed. Then, it is treated with a temporary treatment medium for any of these periods.

These obtained developmental engineering products can be injected into the diseased site of the disease patient, or the like, and used for therapy such as transplantation as at least a part of a sheet, a tissue, or an organ. At this time, the product of the developmental engineering product according to the present embodiment may be transplanted to a patient by preparing a single-layer or multi-layer sheet by using the culture equipment used by those skilled in the art. Further, the cells according to the present embodiment can be cultured by using an appropriate carrier or laminated by using a 3D printer, or the like, to transplant a more organized culture.

That is, the transplantation method according to the present embodiment also functions as the therapeutic method according to the present embodiment.

Further, the product of the developmental engineering according to the present embodiment can be used as a medicine.

In addition, when the transplantation method and the therapeutic method according to the present embodiment are applied to humans among mammals, they should be carried out within the necessary range and limit in accordance with the ethics of various reproductive medicines. That is, it is necessary to avoid genetic modification normally, and even if genetic modification is necessary for the purpose, it should be carried out to the minimum extent according to specific criteria for genetic diseases and prevention of infectious diseases, or the like.

In addition, when the present invention is carried out in Japan, transplantation and treatment after the provision of the culture is performed by a medical doctor. Therefore, the "animal" of the therapeutic method of the present invention does not include humans (Homo sapiens). On the other hand, in other countries, the definitions of "animal" and "therapeutic method" are not limited.

On the other hand, the medicine according to the embodiment of the present invention can also be used for animal treatment for treating animals other than humans. This animal is not particularly limited and includes a wide range of vertebrates and invertebrates. The vertebrates include fish, amphibians, reptiles, birds, and mammals. Specifically, for example, the mammals may be various animals such as the above-mentioned Primates, Rodentia, Lagomorpha, Cetartiodactyla, Perissodactyla, and Carnivora. Specifically, it may be a mouse, a rat, a hamster, a guinea pig, a rabbit, a sheep, a pig, a cow, a horse, a dog, a cat, a ferret, a non-human transgenic primate, or the like. In addition to mammals, wild animals such as fish, birds including poultry, reptiles, and the like, are included. It also broadly includes crustaceans including shrimp and insects, and other invertebrates such as squid, and the like.

That is, the medicine according to the embodiment of the present invention can be used not only for human treatment but also for various animal therapy, livestock growth promotion, and the like.

Further, the medicine according to the embodiment of the present invention can also be a therapeutic target for a part of the body of an animal, or an organ or tissue removed or excreted from the animal. Furthermore, the therapy is a treatment in a broad sense, and it can be applied to a bioreactor, a culture in model animals, a culture of a culturing organ for human transplantation, and the like.

In addition, the developmental engineering product according to the present embodiment can be applied to therapeutic uses other than regenerative medicine, such as bioreactor, production of an artificial organ, creation of a cloned individual, or the like, as for the various uses.

When performing the therapeutic method according to the embodiment of the present invention, the administration interval and dose of the developmental engineering product can be appropriately selected and changed according to various conditions such as the condition of the disease and the condition of the subject, or the like.

The single dose and frequency of administration of the developmental engineering product according to the embodiment of the present invention can be selected and changed as appropriate depending on the purpose of administration and further depending on various conditions such as the age and weight of the patient, the symptoms, the severity of the disease, and the like.

The number and duration of administration may be only once, or once to several times a day for several weeks, the condition of the disease may be monitored, and administration may be repeated or repeated depending on the condition.

In addition, the developmental engineering product of the present invention can be used in combination with other compositions, and the like. Further, the product of the present invention may be administered at the same time as other compositions, or may be administered at intervals, but the order of administration is not particularly limited.

Further, in the embodiment of the present invention, the period for which the disease is improved or alleviated is not particularly limited, but may be temporary improvement or alleviation, or may be improvement or alleviation for a certain period.

### As configured in this way, the following effects can be obtained.

Typically, in order to produce a genetically modified animal, or the like, it is necessary to culture an embryo outside the body and perform embryo manipulation such as modifying the gene, and the like. However, since the embryo cultured and manipulated outside the body is damaged, it is often not possible to produce the individual depending on the lineage or animal species.

For example, when inbred animals are used in the typical method, a large number of fertilized eggs should be used. Specifically, for example, in the electroporation method, a large number of fertilized eggs is required to edit the genome of the inbred strain C57BL/6. Moreover, when a plurality of embryo manipulations outside the body are performed, almost all of them become embryogenesis arrest. That is, the inbred C57BL/6 can only perform one electroporation manipulation. This is because the inbred C57BL/6 has low resistance to damage.

Here, although it has been reported that a mouse having multiple gene mutations, a Flox mouse, a knock-in mouse, and the like, are producible by various methods, the animal species and strains that can be actually produced are limited.

In addition, when a plurality of manipulations is required, a non-inbred strain (BDF1, or the like) that is resistant to damage is used. Specifically, in recent years, many reports have been made on the production of genetically modified animals by genome editing such as CRISPR-Cas as basic research on the production of disease model animals and gene therapy models. However, there are currently very few reports other than specific strains of limited animals (for example, mouse hybrid strains) that are highly resistant to artificial embryo manipulation.

For example, in the 2STEP method, which seems to have the highest damage due to embryo manipulation, no successful case has been reported in pure strain (C57BL/6, or the like) that is widely used in animal experiments.

In addition, even in hybrid mice that are highly resistant to embryo manipulation, many embryos result in maturational arrest or developmental arrest by highly damaged embryo manipulation, and a large amount of fertilized eggs are needed to supply for ensuring the production of the targeted genetically-modified animal.

That is, in the production of typical genetically modified animals, and the like, firstly, it is necessary to select animal species and strains that can withstand culturing and manipulating in-vitro, and further, to use a large amount of these fertilized eggs, and to reduce the damage as much as possible by embryo manipulation.

On the other hand, the temporary treatment medium according to the embodiment of the present invention can reduce damage to embryos, and the like, due to the manipulation of in-vitro-culture, and an individual, an organ, a tissue, a cell and the like, can be efficiently produced.

That is, a fertilized egg, or the like of a strain that is less resistant to manipulation is treated with a temporary treatment medium containing an intracellular skeleton regulator and/or an apoptosis inhibitor that protects against damage and stabilizes at a specific time, it suppresses the developmental arrest and enables the development into blastocysts and individual production. This makes it possible to facilitate embryo manipulation of inbred mice commonly used in experiments, reduce the number of fertilized eggs required for embryo manipulation, and increase the efficiency of individual production. In other words, genetically modified individuals, tissues, organs, cells, and the like, can be obtained from early embryos of animal species and strains that are difficult to obtain large numbers of fertilized eggs and have low resistance to culturing and manipulation in vitro. Therefore, it can be used as a core technology in developmental engineering for producing genetically modified animals, and the like.

Specifically, as shown in the following examples, the temporary treatment medium containing the apoptosis inhibitor according to the present embodiment almost eliminates the embryogenesis arrest that occurs after electroporation, and the number of fertilized eggs required for genome editing in the inbred strain C57BL/6 becomes at a normal range. In addition, Flox mice by the 2STEP method, which requires a plurality of manipulations, with the inbred C57BL/6 can be produced. Furthermore, establishing ES cells and producing individuals by using a heterologous mouse, which has been previously impossible, becomes possible.

That is, by using the temporary treatment medium according to the present embodiment,
1. Individual production becomes possible with a small number of fertilized eggs.
2. A plurality of embryo manipulations in vitro becomes possible.
3. Individuals and cells can be produced from various animal species and special strains by various methods such as gene mutation at multiple loci, Flox, and knock-in.
4. It can also be applied to human assisted reproductive technology.

In addition, in the above-described embodiment and the examples described as follows, an example of efficiently producing an individual as a developmental engineering product from an embryo damaged by culturing and manipulation in-vitro has been described.

Regarding this, it is possible to carry out the embryogenesis arrest inhibitory method, the temporary treatment medium, or the like, according to the present embodiment in order to increase the success rate by using the conventional technique for producing an individual in combination.

Further, in the above-described embodiment, an example in which a low molecular weight compound such as Y-27632 is contained in a temporary treatment medium as a Rho kinase inhibitor has been described.

However, genes, gene products, agonists / antagonists subjected to regulation of Rho-kinase expression, and compositions via action on other pathways can also be used as Rho-kinase inhibitors.

In addition to the above-mentioned temporary treatment medium, cytochalasins, and the like, may be added to the manipulation solution in order to prevent physical damage during manipulations such as cell fusion, nuclear transplantation, injection, and the like.

In addition, the temporary treatment medium according to the embodiment of the present invention can be used in combination with another composition, and the like.

In addition, by using the temporary treatment medium according to the present embodiment, individuals, organs, tissues, cells, or the like, can be produced, efficiently. Furthermore, it is also conceivable to use for gene modification of rare breed, gene therapy for animals with genetic diseases, pet treatment, fertility treatment, and enabling a technology other than Cas9 as shown in the following example.

### [Example 1]

Hereinafter, the method for suppressing embryogenesis arrest and the method for producing a developmental engineering product according to the embodiment of the present invention is described more specifically as examples based on specific experiments. However, the following example is just as one example, and it is not limited thereto.

### [Materials and methods]

### [Reagent]

### (Main reagents)

PZM-5 medium (manufactured by Research Institute for the Functional Peptides: IFP0410P): medium for porcine culture embryo development, PBM medium (manufactured by Research Institute for the Functional Peptides: IFP1030P): medium for late porcine embryo culture, KSOM AA medium (manufactured by the present inventors), mR1ECM medium (manufactured by ARK Resource Co., Ltd.), mWM medium (modified Whiteten's medium) (manufactured by Ark Resource Co., Ltd.), Opti-MEM medium (Product of GIBCO BRL), M2 medium (Product of Sigma, catalog number M7167), Y-27632 (manufactured by Fuji Film Wako: 034-24023).

In addition, IVF (In Vitro Fertilization) -related reagents were obtained from the Research Institute for the Functional Peptides. dbcAMP (Manufactured by SIGMA aldrich: D0627), FSH (Manufactured by Merck Serono: Gonalef 150 for S. C. injection 150), PMSG (Manufactured by Kyoritsu Seiyaku: Serarumon), hCG (Manufactured by Kyoritsu Seiyaku: Gesutoron), TGF-α (Manufactured by R & D systems: 239-A), Pig follicular fluid (prepared by the present inventors. The collected liquid at the time of egg collection is centrifuged with 10000 rpm, 30 min and collected supernatant filtration sterilized liquid).

### (Preparation of other reagents)

### (2 nmol Alt-R CRISPR crRNA)

It was placed in 20 µL of IDTE buffer or Nuclease-free water to make 100 µM. It was dispensed and stored at -80 °C.

### (20 nmol Alt-R CRISPR tracrRNA)

200 µL of IDTE buffer or Nuclease-free water was added to make 100 µM. It was dispensed and stored at -80 °C.

### (CrRNA: tracrRNA stock solution (100 µL))

4.5 µL (final 18 µM) of crRNA (100 µM), 4.5 µL (final 18 µM) of tracrRNA (100 µM), and 16 µL of Nuclease-free Duplex Buffer were prepared. The final amount was 25 µL.

The crRNA and tracrRNA were heated at 95 °C for 5 minutes and hybridized at 15 - 25 °C. For reference, crRNA average molecular weight: 11,700 g/mol and 100 µM = 1.17 µg/µL, tracrRNA molecular weight: 22,182 g/mol and 100 µM = 2.22 µg/µL, crRNA: tracrRNA: 100 µM = 3.39 µg / µL, 1 µM = 33.9 ng / µL.

### (Working solution of electroporation method (10 µL) (CUY21 EDIT))

1.0 µL (final 100 ng / µL) of GeneArt Platinum Cas9 Nuclease (1 µg / µL, Product of Thermo), 1.7 µL (final 3 µM, about 100 ng / µL) of crRNA: tracrRNA stock solution (18 µM), 0.76 µL (final approximately 400 ng / µL) of ssODN (High concentration singlestranded oligodeoxynucleotide) (100 µM), and 16.6 µL of Opti-MEM solution are prepared. This was used as the standard concentration.

### [Equipment]

NEPA21 (registered trademark, manufactured by Nepa Gene Co., Ltd.): Gene transfer device body, CUY501P1-1.5: MS platinum block electrode, 1 mm gap Capacity: 5 µL, C115CB or C115CB-2: Cable (connected to the device body), C117: Cable (connected to the platinum plate electrode via C115CB).

### [Embryogenesis arrest inhibitory method, temporary treatment method, other culture methods]

(Technique for Animal Knockout system by Electroporation, hereinafter also referred to as "TAKE")

The TAKE was carried out according to the method described in Patent Document 2.
1. In the bathtub of the electrode, Cas9 Nuclease, gRNA, ssODN solution, and the like, were put into a genome editing solution or Opti-MEM solution having a volume suitable for knock-out or knock-in.
2. The solution resistance value was adjusted to 200Ω (190 to 210Ω).
3. Firstly, the in-vitro-culture was taken out from the culture solution covered with mineral oil (hereinafter referred to as "drop") and washed once with the Opti-MEM solution applied in the dish.
4. The in-vitro-culture was then loaded into the genome editing solution or Opti-MEM solution in the electrode.
5. The actual resistance value was measured. If the resistance value was 160Ω or less, the genome editing solution or the Opti-MEM solution was slightly aspirated, the resistance value was measured again, and the resistance value was adjusted to around 200Ω (about 190Ω to 210Ω). If the resistance value was 220 Ω or more, the genome editing solution or the Opti-MEM solution was added, the resistance value was measured again, and the resistance value was adjusted to 200 Ω (about 190 Ω to 210 Ω).
6. Immediately after aligning the resistance values, the start button of NEPA21 was pressed to perform electroporation (EP) processing.
7. After the EP treatment, the in-vitro-culture was taken out.

After that, the in-vitro-culture washed with another Opti-MEM solution was put into the liquid in the bathtub, the resistance value was made uniform, and EP was performed in the same manner.

### (In the case of unfrozen inbred strain)

1. By 3 hours before TAKE, Y-27632-added mWM medium (Y-27632 final concentration 10 µM, an example of the "temporary treatment medium" of this example) and Y-27632-free mWM medium (an example of "normal medium" of this example) were dropped on 35 mm Petri dish in 5 drops, respectively, to prepare the drops covered with paraffin oil.
2. Pronuclear stage embryos were collected from the drops, placed in Y-27632-added mWM medium, and cultured for about 30 minutes to 1 hour (temporary treatment).
3. After that, TAKE was performed. At the time of this TAKE, as described above, the pronuclear stage embryo, after washing, was transferred to a genome editing solution for TAKE or an Opti-MEM solution. The same applied to the following processing.
4. After TAKE, the embryos were placed in Y-27632-added mWM medium (temporary treatment) and cultured under 37 °C, 5% CO₂, and (5% OO₂) conditions until the next day (1 hour or more and within 12 hours).
5. If necessary, culture was continued or embryo transfer was performed in Y-free mWM medium.

### (For standard frozen inbreeds or special strains)

1. Up to 3 hours before TAKE, Y-27632-added mWM medium (Y-27632 final concentration 10 µM, an example of the "temporary treatment medium" of this example) and Y-27632-free mWM medium (an example of "normal medium" of this example) were dropped into 35 mm Petri dishes by 5 drops, respectively, to prepare the drops covered with paraffin oil.
2. Pronuclear stage embryos were collected from the drops, placed in Y-27632-added mWM medium, and cultured for about 30 minutes to 1 hour (temporary treatment).
3. After that, TAKE was performed.
4. After TAKE, the embryos were placed in Y-27632-added mWM medium and cultured for about 30 minutes to 1 hour (temporary treatment).
5. Then, the embryos were placed in Y-free mWM medium and cultured at 37 °C under 5% CO₂ and (5% O₂) conditions until the next day. If necessary, culture was continued or embryo transfer was performed in Y-27632-free mWM medium.

### (Inbred strains derived from frozen and unfrozen heterologous wild mice)

1. Up to 3 hours before TAKE, Y-27632-added mWM medium (Y-27632 final concentration 10 µM, an example of the "temporary treatment medium" of this example), Y-27632-free mWM medium (an example of "normal medium" of this example) were dropped into 35 mm Petri dishes by 5 drops, respectively, to prepare drops covered with paraffin oil.
2. Two-cell stage embryos were collected from the drops or thawed, placed in Y-27632-added mWM medium, and cultured for about 30 minutes to 1 hour (temporary treatment).
3. After that, TAKE was performed.
4. After TAKE, the embryos were placed in Y-27632-added mWM medium and cultured for about 30 minutes to 1 hour (temporary treatment). Then, the embryos were placed in Y-27632-free mWM medium and cultured at 37 °C under 5% CO₂ and 5% O₂ conditions until transplantation.
5. Then, if necessary, the culture was continued in the Y-27632-free mWM medium to establish ES cells.

### (For pig IVM and IVF embryos)

1. On the day before TAKE, 5 drops of Y-27632-added PZM-5 medium (Y-27632 final concentration 10 µM, an example of the "temporary treatment medium" of this example) was dropped on a 35 mm Petri dish to prepare a drop coated with paraffin oil. In the drop of this example, 20 µL of medium was added dropwise, 1 mL of oil was added, then 20 µL of medium was added again, and the mixture was completely covered with oil. This drop produced twice as many as the TAKE.
2. IVF was performed based on a method common to those skilled in the art.
3. On the day after fertilization, IVF embryos were placed in Y-27632-added PZM-5 medium and cultured for about 30 minutes to 1 hour (temporary treatment).
4. After that, TAKE was performed.
5. The embryos were returned to Y-27632-free PZM-5 medium (an example of the "normal medium" of this example) and cultured under the conditions of 39 °C, 5% CO₂, and 5% O₂.
6. If necessary, sampling, or the like, was performed at the development stage. If the culture is to be continued until the blastocyst stage, the drops containing Y-27632-free PZM-5 medium and PBM medium was made in the same way as the step of "1." at four days after insemination.
7. Five days after the insemination, the embryos that had developed up to the morula stage and the blastocyst stage were transferred to PBM medium (an example of the "normal medium" of this example). The remaining surviving embryos were also transferred to Y-27632-free PZM-5 medium, and when development progressed, they were appropriately transferred to PBM medium.
8. Blastocysts to expanded blastocysts were formed 7 to 8 days after insemination.

### (Inbred mouse 2STEP method)

1. Up to 3 hours before TAKE, Y-27632-added mWM medium (Y-27632 final concentration 10 µM, an example of the "temporary treatment medium" of this example), Y-27632-free mWM medium (an example of "normal medium" of this example), 5 drops of each medium were dropped on 35 mm Petri dish, and then covered with paraffin oil. For the drops, twice the number of the drops plates to be performed TAKE were prepared.
2. Pronuclear stage embryos were collected, placed in Y-27632-added mWM medium, and cultured for about 30 minutes to 1 hour (temporary treatment).
3. After that, the first TAKE was performed.
   3a. In the case of frozen eggs, after the first TAKE, they were placed in a Y-27632-added mWM medium and cultured for about 30 minutes to 1 hour (temporary treatment).
   3b. In the case of unfrozen eggs, after the first TAKE, they were placed in a Y-27632-added mWM medium and cultured under 37 °C and 5% CO₂ (5% O₂) conditions until the next day (temporary treatment).
4. After the temporary treatment, the embryos were placed in Y-27632-free mWM medium and cultured at 37 °C and 5% CO₂ (5% O₂) conditions until the next day.
5. Both were confirmed at the next day, and the embryos at the 2-cell stage were subjected to the second TAKE.
6. After the second TAKE, the embryos were placed in Y-27632-added mWM medium and cultured for about 30 minutes to 1 hour (temporary treatment).
7. Then, they were transferred to Y-27632-free mWM medium. If necessary culture was continued or embryo transfer was performed.

### (Inbred rat 2STEP method)

1. At least 3 hours before TAKE, 5 drops ofY-27632-added KSOM AA medium (Y-27632 final concentration 10 µM, an example of the "temporary treatment medium" of this example) were placed on a 35 mm Petri dish and drops covered with paraffin oil.
2. Pronuclear stage embryos were collected, placed in Y-27632-added KSOM AA medium, and cultured for about 30 minutes to 1 hour (temporary treatment).
3. After that, the first TAKE was performed.
4. After TAKE, the embryos were placed in Y-27632-added KSOM AA medium and cultured under 37 °C, 5% CO₂, and 5% O₂ conditions for 30 minutes and more and up to 12 hours (temporary treatment).
5. Then, they were placed in Y-27632-free KSOM AA medium (an example of the "normal medium" of this example), and cultured under 37 °C, 5% CO₂, and 5% O₂ conditions until the second TAKE.
6. The next day, the embryos that had become two cells were separated, and about 20 to 22 hours after egg collection, they were placed in Y-27632-added KSOM AA medium again and cultured for about 30 minutes to 1 hour (temporary treatment).
7. After that, the second TAKE was performed.
8. After the second TAKE, the embryos were placed in Y-27632-added KSOM AA medium and cultured for about 30 minutes to 1 hour (temporary treatment).
9. Then, they were transferred to mR1ECM medium (an example of "normal medium" of this example). If necessary, culture was continued or embryo transfer was performed.

### (Embryo transfer)

According to a typical method, in the afternoon when female mice and rats were mated with males with vasoligation and confirmed to be plugged, about 10 each were transplanted into the left and right oviducts or uterus of plug-confirmed mature female mice and rats.

In the case of pigs, about 10 embryos each were transplanted into the uterus by a non-surgical method.

### [result]

### [Overall summary]

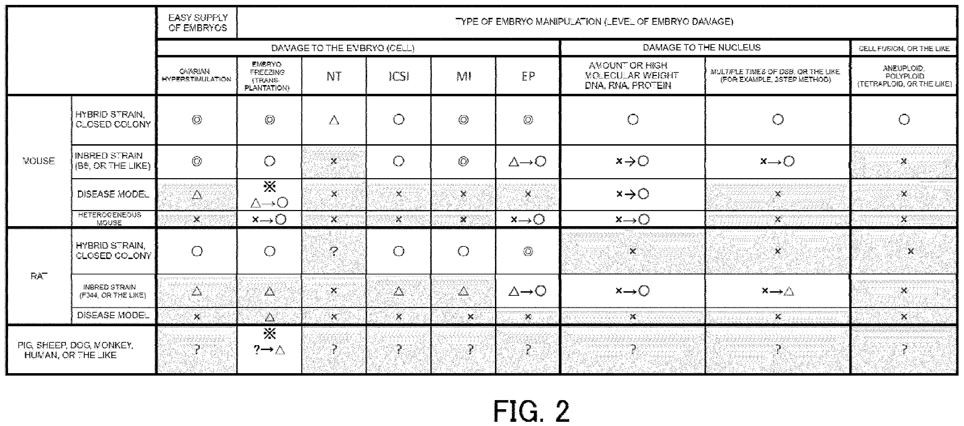

Firstly, with reference to FIG. 2, a strain capable of producing an individual and a manipulation by the embryogenesis arrest inhibitory method and the developmental engineering product production method of the present example are described.

In FIG. 2, in the mouse, the difficulty of creating an individual of a hybrid, a closed colony, an inbred (B6, or the like), a disease model, and a heterologous mouse, is shown. In the rat, the difficulty of creating individual of a hybrid, a closed colony, an inbred (F344, or the like), and a disease model is shown. In pigs, sheep, dogs, monkeys, and humans, the difficulty in some treatments is also shown. In addition, for each manipulation, as manipulations that damage embryos (cells), ovarian hyperstimulation, frozen embryo (transfer, embryo freezing), nuclear transplantation (NT), intracytoplasmic sperm injection (ICSI), microinjection (MI), electroporation method (EP) are shown for respective manipulation. Furthermore, as damage to the nucleus, exposure to a large amount or high molecular weight DNA and a manipulation involving multiple DSBs (2STEP method, or the like) are shown. Further, the manipulation of making aneuploids and polyploids (tetraploids, or the like) with cell fusion, and the like, is shown.

Specifically, regarding the creation of an individual, the symbols in each column indicate the difficulty level: a double circle to (easy, low difficulty), a single circle to (possible, normal difficulty), a triangle to (high difficulty), a cross mark to (impossible). That is, it shows the difficulty in the order of the double circle, the single circle, the triangle, and the cross mark. The question mark indicates that the difficulty level is unknown.

Here, the degree of difficulty indicated by the tip of the arrow in each column shows what the inventors have actually demonstrated to be able to produce by the embryogenesis arrest inhibitory method and the developmental engineering product production method of this example. The gray (dark) backgrounds in each column indicate the extents in which the effects of this example can be expected, but there are no test examples yet.

As described above, it is possible to utilize the embryogenesis arrest inhibitory method and the developmental engineering product production method of this example for a wide range of mammals, strains, and manipulations.

Hereinafter, a treatment example of each in-vitro-culture as shown in FIG. 2 is described.

### (Treatment example 1)

Firstly, an example of producing a knockout mouse by an electroporation method by using a C57BL/6J mouse is described.

Typically, fertilized eggs treated by the electroporation method (TAKE) usually had a low development rate and a low birth rate, so it was necessary to increase the number of transplanted embryos per recipient.

In this production example, the purpose was to increase the development rate and the birth rate by adding Y-27632 and reduce the number of transplanted embryos.

Therefore, in this production example, temporary treatment was performed with a temporary treatment medium containing Y-27632 before and after TAKE. Pronuclear stage embryos of C57BL/6J (CLEA Japan, Inc.) were used as in-vitro-culture and female MCH mice (CLEA Japan, Inc.) were used as recipient.

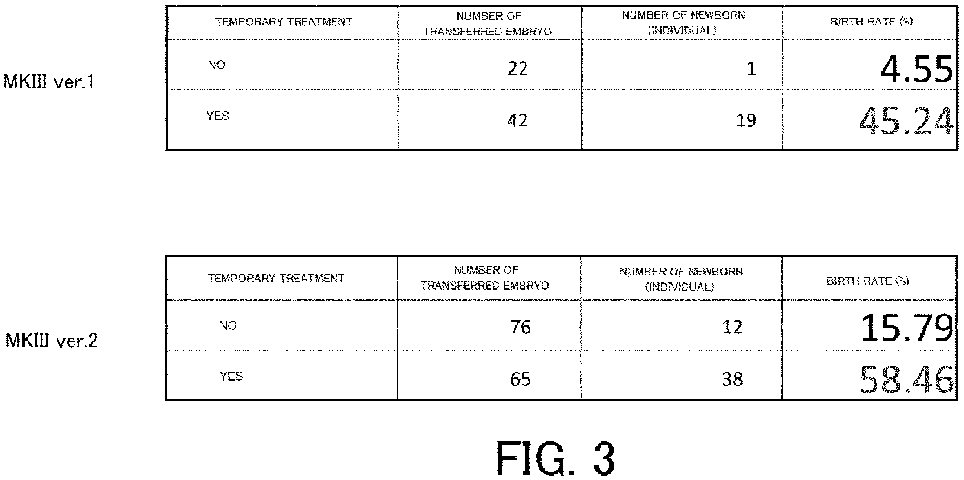

According to FIG. 3, as a result, before and after electroporation, the embryos were cultured in a culture medium containing Y-27632 as an example of the intracellular skeleton regulator and/or apoptosis inhibitor according to the present embodiment. When the Mk-3 gene was knocked out with two types of gRNA ("MKIII ver. 1" or "MKIII ver. 2"), the birth rate increased about 5 times. At this time, the the efficiency of nonhomologous end joining (NHEJ) did not change.

### (Treatment example 2)

Next, an example of producing a Flox mouse by the 2STEP method by using C57BL/6J is described.

Typically, inbred Flox mice cannot be obtained from inbred fertilized eggs treated with the 2STEP method because the development rate and the birth rate are remarkably low. Therefore, in this production example, the purpose was to increase the development rate and the birth rate and to produce an inbred Flox mouse.

Therefore, in this preparation example, the temporary treatment medium containing Y-27632 was temporarily treated before and after the electroporation (EP) of the 2STEP method. Pronuclear stage embryo of C57BL/6J (CLEA Japan, Inc.) were used as an in-vitro-culture and female MCH (CLEA Japan, Inc.) were used as recipient.

The present inventors conducted an experiment to obtain Flox mice by the 2STEP method targeting the Mecp2 gene.

The results are shown in Table 2 below:

**[Table 2]**

| EXPERIMENTAL GROUP | Left loxP | Right loxP | 2loxP |
|---|---|---|---|
| m→m | 5/23 (21.7%) | 2/23 (8.7%) | 0/23 (0%) |
| m→Y | 1/17 (5.9%) | 1/17 (5.9%) | 0/17 (0%) |
| Y→Y | 1/15 (6.7%) | 2/15 (13.3%) | 1/15 (6.7%) |

| | | | |
|---|---|---|---|
| Target: Mecp2-flox | | | |

In Table 2, "m" indicates treatment with the normal medium, and "Y" indicates temporary treatment with the temporary treatment medium. Each experimental group for the first electroporation step and the second electroporation step of the 2STEP method indicates that it is treated with the normal medium (m) or the temporary treatment medium (Y), respectively. That is, "m -> m" uses the normal medium in the both step, "m -> Y" was temporarily treated with the temporary treatment medium only in the first electroporation step, and "Y -> Y" was temporarily treated with the temporary treatment medium in both steps.

In each column, the proportions that the loxP sequence is inserted only on the left side by the first electroporation step (Left loxP), the loxP sequence is inserted only on the right side by the second electroporation step (Right loxP), and both loxP sequences inserted by both steps (2loxP) were respectively shown.

As a result, 1 in 15 blastocysts (7%) and 1 in 25 neonates (4%) of C57BL/6J Flox mice were obtained. On the other hand, in the experimental group to which Y-27632 was not added, neither blastocyst nor newborn was obtained.

### Similarly, in the Drb1 gene, newborn C57BL/6J Flox mice were obtained.

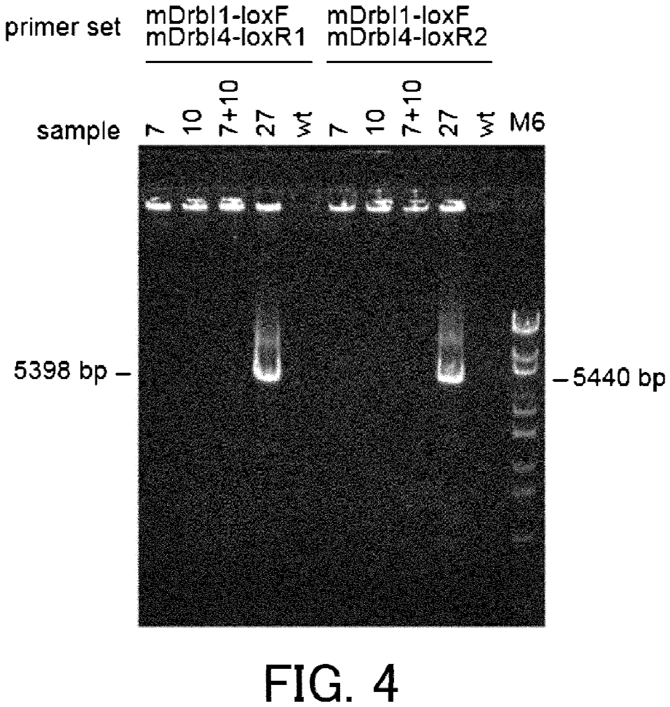

FIG. 4 shows an example of a Flox mouse, which is able to be conditional knockout with this 2loxP. In the "27" lane, bands (5398 bp and 5440 bp) indicate that it is a Flox mouse.

In this way, by the 2STEP method, we tried to produce inbred Flox mice that can be knocked out conditionally by using two types of genes. Previously unreported inbred strains of Flox mice were obtained only from the experimental groups to which Y-27632 were added. As described above, the temporary treatment by Y-27632 is considered to be effective for a strain vulnerable to damage such as B6, or the like.

### (Treatment example 3)

Then, an example of individual production from frozen eggs of an inbred strain SPR2 of a heterologous mouse, Mus spretus (Algerian mouse) (hereinafter, simply referred to as "heterogeneous inbred SPR2 mouse" or simply "SPR2"), and a treatment example in which ES cells were established from frozen embryos of the heterologous mouse are described.

In this production example, two-cell frozen embryos of SPR2 were thawed, cultured in a temporary treatment medium containing Y-27632 for 1 hour, and transplanted into the uterus of a pseudopregnant female mouse.

In this example, two-cell frozen embryos of heterologous inbred SPR2 mouse were thawed, cultured for 1 hour in a culture medium containing Y-27632, and transplanted into the uterus of a pseudopregnant female mouse. As a result, frozen embryo-derived individuals (16 transplanted embryos, 6 newborns) were obtained.

The results are shown in Table 3 below:

**[Table 3]**

| SPR2 TRANSPLANT RESULTS | | | | | |
|---|---|---|---|---|---|
| EMBRYO TRANSFER STAGE | TRANSPLANTATION SITE | NUMBER OF EMBRYOS TRANSFERRED | NUMBER OF BIRTHS | | TOTAL |
| | | | MALE | FEMALE | |
| BLASTOCYST | UTERUS(3.5DAYS) | 16 | 2 | 4 | 6 |

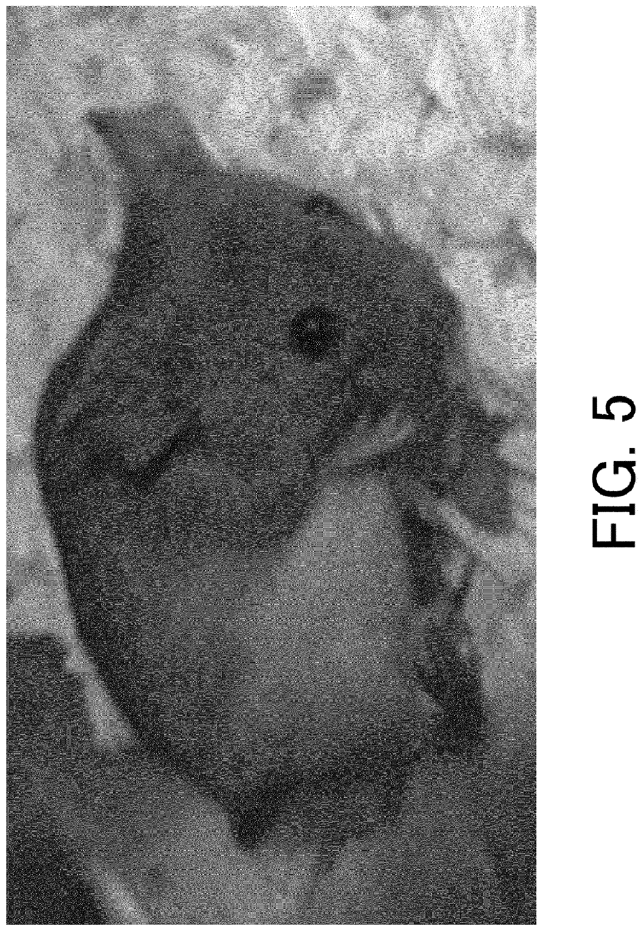

FIG. 5 shows an example of the obtained individual. Individual creation from frozen embryos in heterologous inbred SPR2 mouse has not been possible, conventionally.

Then, the results of a treatment example of ES cell establishment from heterologous inbred SPR2 mouse frozen embryos is described.

After thawing a two-cell frozen embryo of a heterologous inbred SPR2 mouse, it was cultured to a blastocyst in the temporary treatment medium containing Y- 27632, and an attempt was made to establish ES cells. As a result, ES cells could be efficiently established (5 frozen embryos, 2 strains established). On the other hand, when Y-27632 was not added, no individual production and ES cells were established.

As described above, the temporary treatment of Y-27632 made it possible to produce ES cells from frozen embryos of heterologous mice, which was not possible, conventionally.

### (Treatment example 4)

Then, an example of treatment in which ES cells were established from early embryos of electroporated heterologous inbred SPR2 mouse and heterologous mouse Mus caroli (Ryukyu mouse) is described.

Early embryos of the heterologous inbred SPR2 mouse and the heterologous mouse Mus caroli were cultured in the medium containing Y-27632, then electroporated to attempt genome editing, and ES cells were established at a high rate. When Mus caroli ES cells were knocked out of the Tyr gene by genome editing and a part of them was analyzed, it was shown that NHEJ occurred and heterologous mouse ES cells were established.

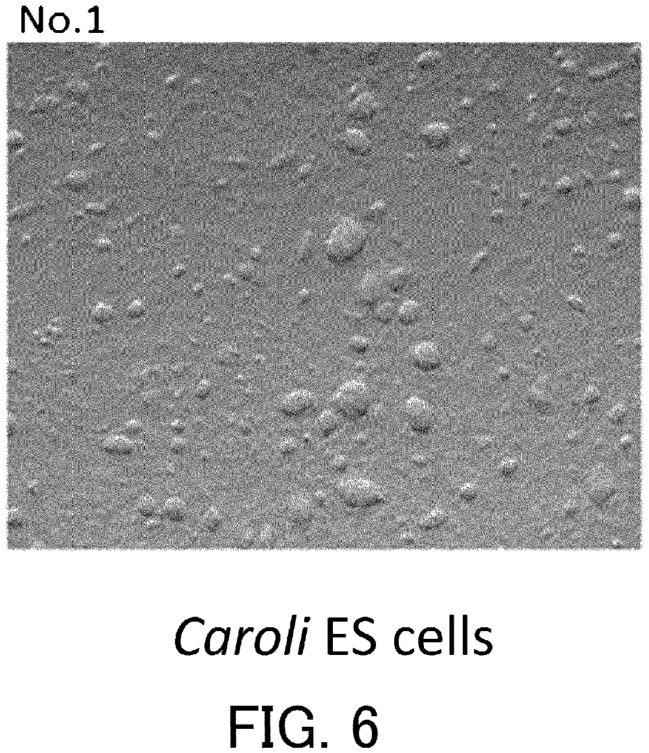

FIG. 6 shows a dish of ES cells established from Mus caroli. The morphology of ES cells can be visually confirmed.

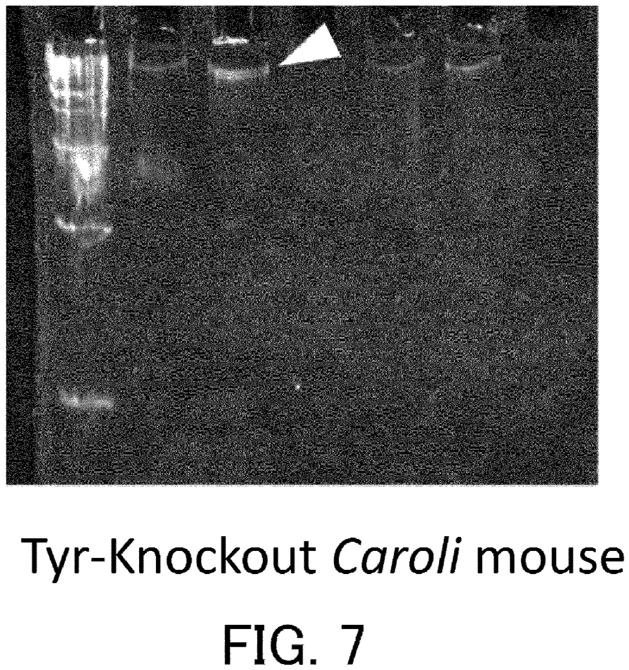

FIG. 7 shows a Mus caroli in which the Tyr gene has been knocked out. The arrowhead is a band that indicates knockout of the gene.

### (Treatment example 5)

Then, an example in which it is verified whether the temporary treatment medium of this example can reduce the adverse effect on the generation of electroporation is described.

Firstly, we conducted experiments on the effects on post-electroporation development by using inbred rat embryos, or the like.

The inbred rat F344 undergoes two electroporation to cause embryogenesis arrest. Regarding this, the results were confirmed by culturing embryos in the temporary treatment medium according to the present embodiment for a specific period before and after electroporation.

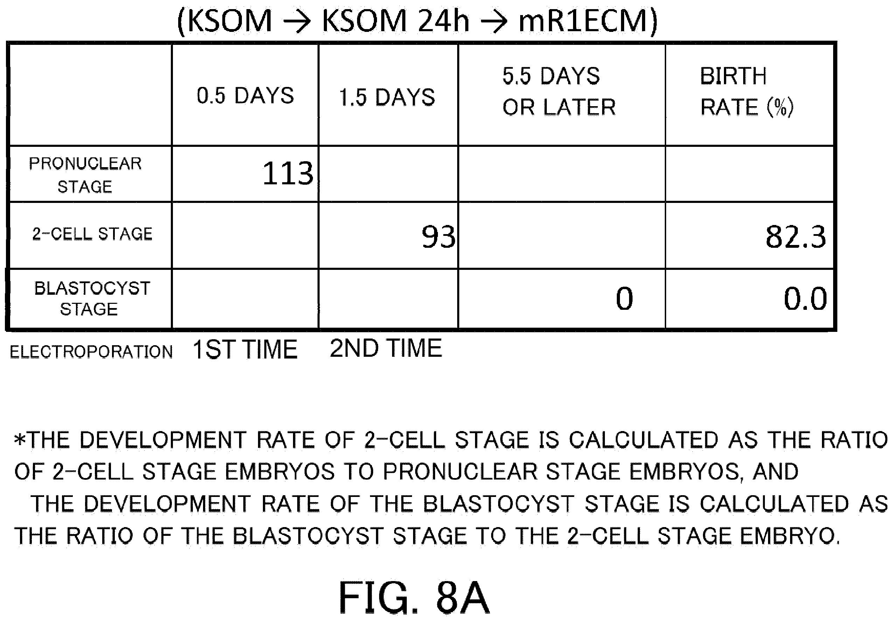

FIG. 8A showed the results of the control that was not treated with the temporary treatment medium according to the present embodiment. None of them even developed into blastocysts.

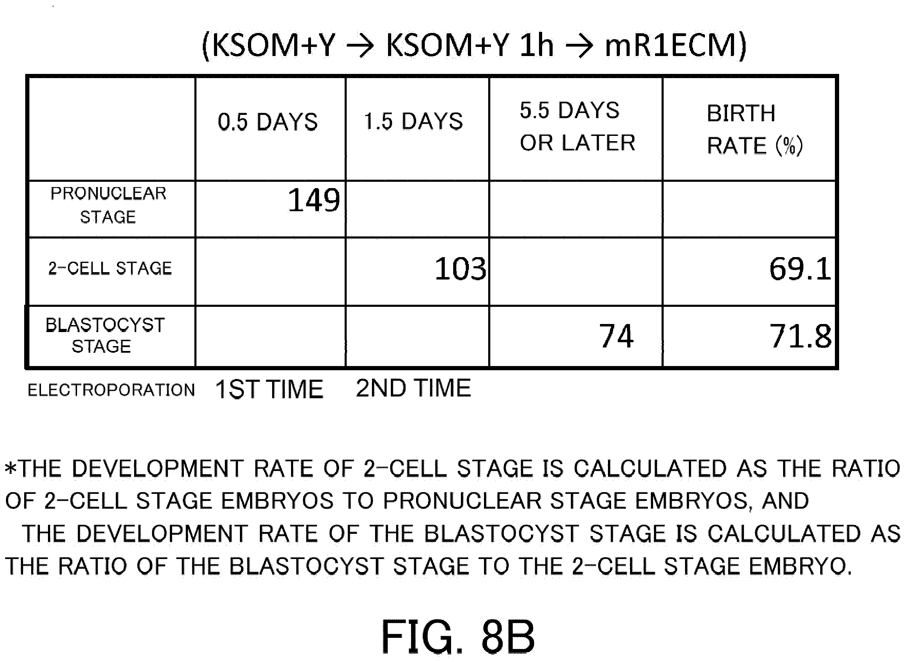

FIG. 8B showed the results of treatment with the temporary treatment medium according to the present embodiment before and after the electroporation. 71.8% of embryos was developed to the blastocyst.

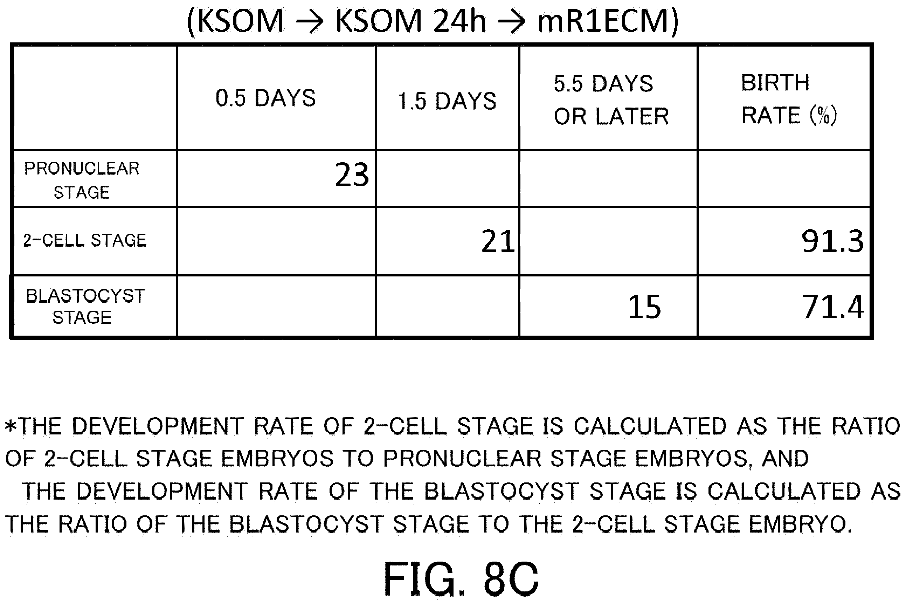

FIG. 8C showed controls that were not electroporated and were not treated with the temporary treatment medium according to the present embodiment. 71.4% of embryos was developed to the blastocyst.

In these figures, the development rate of the two-cell stage was calculated as the ratio of the two-cell stage embryo to the pronuclear stage embryo, and the development rate of the blastocyst stage was calculated as the ratio of the blastocyst stage to the two-cell stage embryo.

As a result, inbred rat F344 was treated with the temporary treatment medium according to the present embodiment before and after electroporation to develop blastocysts without arresting embryogenesis. Specifically, 74 of 103 (71.8%) 2-cell stage embryos developed until the blastocyst stage.

This ratio was about the same as that without treatment and without electroporation.

Next, the effect of the temporary treatment medium of this example on the heterologous inbred SPR2 mouse, which cannot obtain the individual by embryo transfer after freeze-thaw and electroporation, was measured.

The results were shown in Table 4 below:

**[Table 4]**

| TRANSPLANTED EMBRYO INFORMATION | | | | | | TRANSPLANTED SITE | BIRTH METHOD | NUMBER OF BIRTHS |
|---|---|---|---|---|---|---|---|---|
| TRANSFER STRAIN | FERTILIZATION METHOD | FREEZING OR NOT | INTRODUCTION METHOD | EMBRYOSTAGE | NUMBER OF TRANSFERRED EMBRYOS | | | |
| SPR2← ①TAKE ① | NATURAL | YES | TAKE (PULSE 7 TIMES) | 2cell | LEFT)9 RIGHT)8 | OVIDUCT | NATURAL | 7 NEWBORNS |
| M.spretus × Mspretus | | | | | | | | |
| SPR2← ①TAKE ② | NATURAL | YES | TAKE (PULSE 7 TIMES) | BLASTOCYST | LEFT)9 RIGHT)7 | UTERUS (3.5 DAYS) | NATURAL | 5 NEWBORNS |
| Mspretus × M.spretus | | | | | | | | |

In the heterologous inbred SPR2 mouse, the thawed 2-cell stage embryos that were cultured in the temporary treatment medium of this example for the specific period before and after electroporation were used, 17 of 2-cell stage embryos were performed transplant into the oviduct, and 7 individuals were able to be obtained.

Furthermore, 16 embryos (blastocysts) developed in mWM medium up to the blastocyst stage were transplanted into the uterus 3.5 days after confirmation of the plug, and 5 individuals could be obtained.

In addition, the effect of the temporary treatment medium of this example was measured on pig fertilized embryos that had undergone in vitro maturation and in vitro fertilization with frozen semen.

As a result, when cultured in the temporary treatment medium of this example, 35 out of 123 embryos at the 2-cell stage developed into late blastocysts (dilated or prolapsed blastocysts). On the other hand, no blastocysts was obtained when the embryos were cultured only in the control normal medium and electroporated.

### (Treatment example 6)

Then, an example of producing a Flox rat by Knock-In (KI) by electroporation by using a high concentration ssODN in an inbred rat F344 is described.

In the case of electroporating inbred F344 rats, the development has been arrested when the concentration of ssODN and the number of translation pulses were increased. Furthermore, the development was arrested when electroporation was performed twice even at the normal concentration of ssODN and condition. In inbred F344 rats, we verified whether the LoxP sequence, which is not knocked-in when the concentration of ssODN and the number of translation pulses were increased, is possible to be knocked-in and whether 2STEP Flox is possible by culturing in the temporary treatment medium of this example before and after electroporation.

In this production example, knock-in by the 2STEP method was performed on pronuclear stage embryos of F344 rat. The electroporation was performed with ssODN of 800 ng / µL, which is twice the normal concentration, and 14 transfer pulses, which is about twice the normal. For the specific period before and after electroporation, the embryos were cultured in the temporary treatment medium of this example.

The results are shown in Table 5 below:

**[Table 5]**

| | | |
|---|---|---|
| NUMBER OF SAMPLES: | **86** | |
| PCR AMPLIFICATION: | **67** | |
| **Left loxP KI:** | **19/67 (28.4%)** | (PREVIOUS TIME **0/12, 0%**) |
| **Flox:** | **1/67 (1.5%)** | |

Among the 86 samples in the table, 67 samples that has been developed to the blastocysts were obtained, and those in which the Left LoxP sequence was knocked in were first examined by PCR. Thereupon, although LoxP could not be knocked in when treated with the normal medium alone (0 / 12, 0%), the blastocysts were confirmed when treated with the temporary treatment medium of this example (19 / 67, 28%). In addition, they developed to the blastocysts without arresting development, and Flox having loxP sequences at both ends of the target gene was confirmed in the blastocysts (1 / 67, 1.5%).

That is, the LoxP sequences that were not knocked in at the normal ssODN concentration and the number of pulses could be knocked in by culturing them in the culture medium containing Y-27632 before and after electroporation. At this time, it became possible to double the concentration of ssODN during electroporation and double the number of electroporations.

### (Treatment example 7)

Then, an example of producing a multi-gene knockout (Knock-Out, KO) mouse from a small number of fertilized embryo from a mouse having difficulty in obtaining a large number of fertilized eggs such as a disease model mouse, or the like, is described.

In this production example, a multi-gene KO mouse was prepared by using an immunodeficient mouse (nude, a nude mouse) embryo.

Specifically, a male of BALB/c-nu/nu and a female of BALB/c-nu/+ treated with ovarian hyperstimulation were crossed, and a pronuclear stage embryos were collected. The guide RNAs (crRNA: tracrRNA), which are two for the BBOX1 gene and two for the IL2RG gene, are electroporated at about 100 ng / µL per guide RNA, totaling about 400 ng / µL, are electroporated with 14 transfer pulses, and, before and after the electroporation, the embryos were cultured in the temporary treatment medium of this example.

As a result, 63 embryos were transplanted and 16 pups were born. Analysis of some (4 surviving newborn and 3 stillborn newborn) revealed mutations in 3 of them, and 1 of them was confirmed to have mutations in 2 genes. The surviving newborns # 2 and # 4 in Table 6 below are successful examples.

**[Table 6]**

| sample | | delivery | sex | punch | mBBOX1 gene | mBBOX1 mt protein | mlL2RG gene (on X chr) | mlL2RG mt protein |
|---|---|---|---|---|---|---|---|---|
| #1 | nude | 191030 | 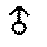 | right ear | wt/wt | (wt: 387aa) | wt | (wt: 369 aa) |
| #2 | nude | 191030 | 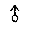 | left ear | wt/-20bp | 149 aa(N-term 148 aa is the same as wt) | -3bp | 368 aa (Pro138del) |
| #3 | white | 191030 | ♀ | right ear | wt/wt | | wt/wt | |
| #4 | white | 191030 | ♀ | left ear | wt/wt | | -2bp/(-2bp+1bp) | 165 aa/145 aa (N-term 138 aa is same as wt) |
| #1 | dead | 191030 | | | wt/wt | | wt/(wt) | |
| #2 | dead | 191030 | | | wt/wt | | wt/(wt) | |
| #3 | dead | 191030 | | | wU-11 bp | 152 aa (N-term 150 aa is the same as wt) | wV(wt) | |

As a result, in nude mice, the individual in which both BALB/c mBBOX1 and mIL2RG genes are knocked out could be produced. On the other hand, in the control treated only with the normal medium, no individual was born in the first place.

### (Treatment example 8)

Next, an example of producing a heterologous knockout (KO) mouse is described.

By using a two-cell stage embryo of a heterologous inbred SPR2 mouse, four guide RNAs (crRNA: tracrRNA) for the Tyr gene were used about 100 ng / µL per RNA, for a total of about 400 ng / µL, and it was electroporated once or twice. Before and after electroporation, the embryos were cultured in the temporary treatment medium of this example.

The results are shown in Table 7 below:

**[Table 7]**

| TRANSPLANTED EMBRYO INFORMATION | FREEZING OR NOT | EMBRYO MANIPULATION | INTRODUCING GENE | NUMBER OF EMBRYOS TRANSFERRED (L, R) | BIRTH DATE | BIRTH METHOD | REMARKS |
|---|---|---|---|---|---|---|---|
| SPR2 × SPR2 | UNFROZEN EMBRYO | TAKE **×1** | Tyr 124ab | 20 (10, 10) | 190401 | NATURAL DELIVERY | SPOTTED:3, BROWN:4 |
| SPR2 × SPR2 | UNFROZEN EMBRYO | TAKE×**2** | Tyr 124ab | 13 (7, 6) | 190717 | NATURAL DELIVERY | SPOTTED:3, BROWN:1, WHITE:3 |
| SPR2 × SPR2 | FROZEN EMBRYO | TAKE**×2** | Tyr 124ab | 16(8,8) | 190726 | CESAREAN SECTION | 1 NEWBORN (WHITE) RESUSCITATION -> EATEN DEATH AS FOSTER CHILD |

As a result, when electroporated only once, 20 embryos were transplanted and 7 individuals were obtained. Among them, there were 3 mosaics. When electroporated twice, 13 embryos were transplanted and 7 individuals were obtained. Among them, 3 mosaics and 3 whites (homo or compound heterozygote) were obtained.

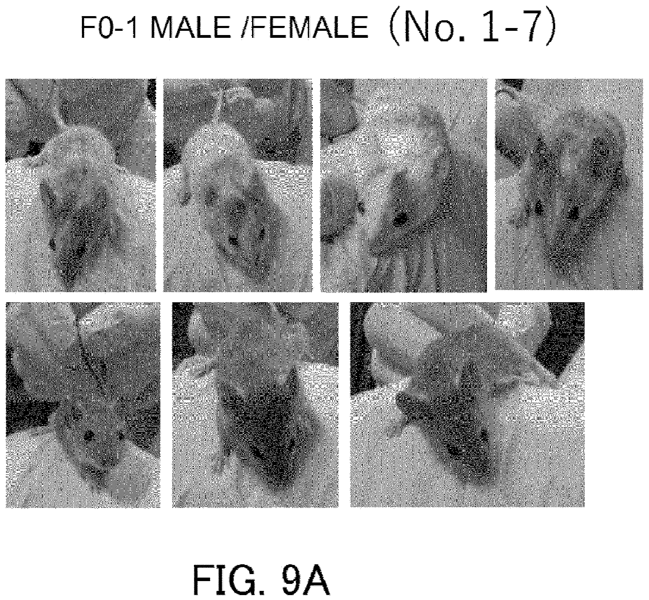

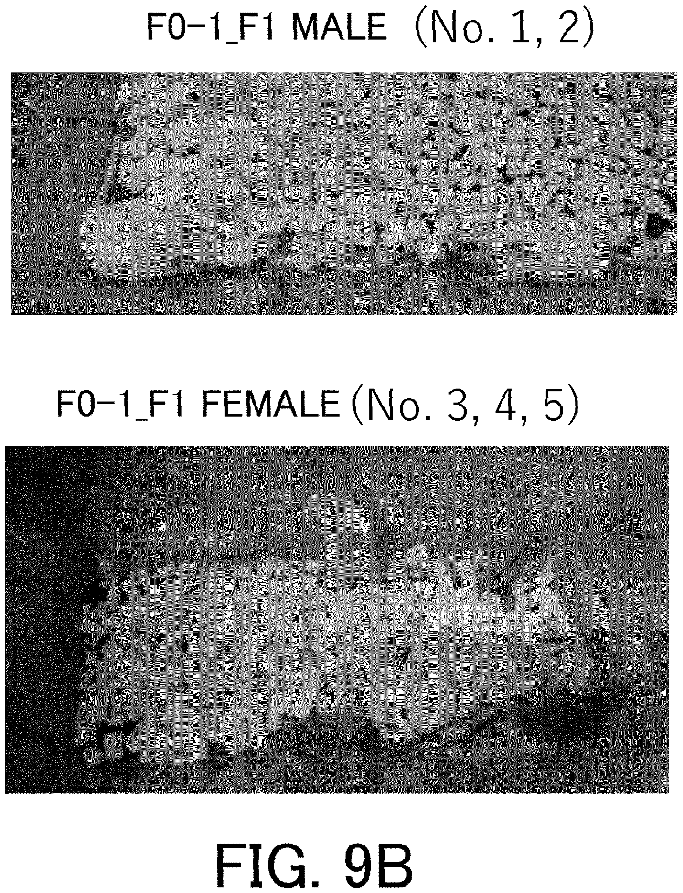

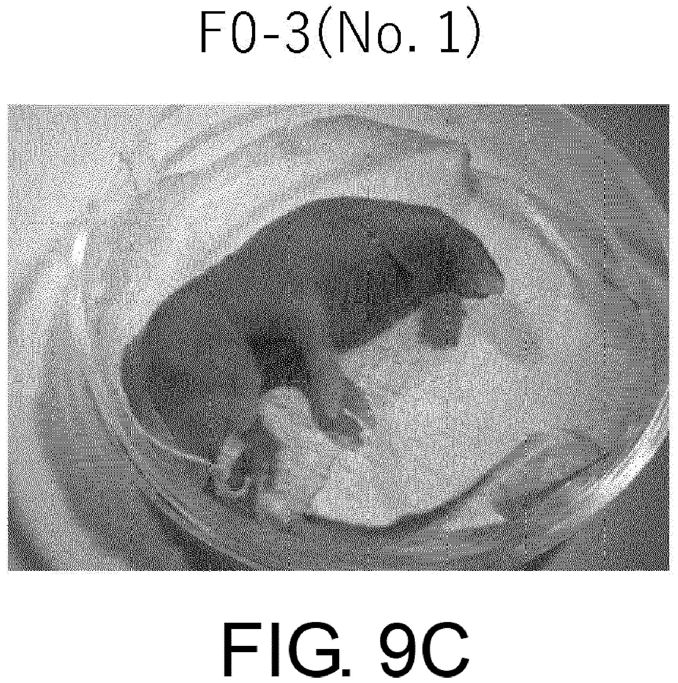

FIGS. 9A, 9B and 9C show examples of the obtained individuals.

As described above, we succeeded in producing a knockout mouse in which the Tyr gene was knocked out from a two-cell frozen embryo of a heterologous inbred SPR2 mouse. That is, KO homozygotes (including compound heterozygote) could be efficiently produced by using inbred strains of heterologous mice.

### (Treatment example 9)

Then, in the early embryos of the inbred mouse B6J strain, an example of using frozen embryos preserved at the blastocyst stage, using the normal medium or the temporary treatment medium according to the present embodiment before and after the manipulation, and comparing whether the occurrence changes is described.

In this treatment example, as the temporary treatment medium, a Y-27632-added mWM medium so as to be 10 µM was used (in this example, it is referred to as "Y+ medium"). In addition, as the normal medium, an mWM medium to which Y-27632 was not added was used (In this example, it is referred to as "Y- medium").

As a treatment, the prepared frozen embryos were thawed and developed as they were in Y- medium until the blastocyst stage.

Then, half of the embryos that had developed up to the blastocyst were transferred to Y+ medium and placed for 30 minutes. After that, the embryos were transferred to Y-medium again and placed for 30 minutes.

Then, the embryos treated with Y+ medium and the embryos not treated were frozen in one tube, respectively.

Both tubes of embryos were then thawed and placed in the normal medium for 10 minutes.

Then, the embryos were evenly divided into Y+ medium and Y- medium, respectively, and placed for 1 hour.

Then, again, each tube of embryos was returned to Y- medium in each tube and the embryos were observed. The observation was performed immediately after thawing, 3 hours later, and 24 hours later.

The results of these manipulations and processes are shown in Table 8 below.

**[Table 8]**

| COMPARING NUMBER OF SURVIVING EMBRYOS AFTER THAWING | | | | | |
|---|---|---|---|---|---|
| CULTURE MEDIUM USED | NUMBER OF EMBRYOS USED | IMMEDIATELY AFTER THAWING | 3 HOURS AND A HALF AFTER THAWING | 24 HOURS AFTER THAWING | RECOVERY RATE |
| Y- 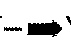 Y- | 19 | 2 | 1 | 8 (1) | 42% |
| Y- 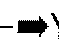 Y+ | 20 | 3 | 5 | 11 (3) | 55% |
| Y+ 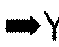 Y- | 26 | 6 | 11 | 14 (7) | 70% |
| Y+ 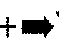 Y+ | 26 | 7 | 12 | 24 (11) | 92% |

| | | | | | |
|---|---|---|---|---|---|
| 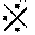 ( ) NUMBER OF OUTGROWTH | | | | | |

As a result of comparing the Y- medium and the Y+ medium, it was possible to confirm that the medium by using the Y+ medium recovered more viable embryos. The recovery rate of embryos by using Y+ medium before and after freezing resulted the best, and the next best condition was that Y+ medium was used before freezing and Y-medium was used after freezing. The results got worse in the following order; Y-medium and Y+ medium were used before and after freezing, and Y- medium was used alone. Although each embryo was frozen twice, it was able to recover up to 92% when developed in Y+ medium.

### (Treatment example 10)

Then, an example in which it was verified whether or not cytochalasin B (hereinafter referred to as "CB"), which was a substance other than Y-27632, as an intracellular skeleton regulator had the same effect, was described.

In this treatment example, as in the above-mentioned treatment example 9, the normal medium (hereinafter, referred to as "Y- medium" as in the treatment example 9), the temporary treatment medium to which Y-27632 is added (hereinafter, referred to as "Y+ medium" as in the treatment example 9), and a CB-added medium (hereinafter, referred to as "CB+ medium") were used. The concentrations of CB in the CB+ medium were 5 µM and 10 µM are prepared. As the manipulation, the recovery rates when frozen embryos were thawed and developed, then re-frozen in blastocysts and re-thawed were compared.

For the treatment, 107 frozen embryos of C57BL/6J (provided from CLEA Japan, Inc.) at the 2-cell stage were used. Firstly, the frozen embryos at the 2-cell stage were thawed and blastocysts were developed as they were in a normal medium.

Then, the blastocysts were then transferred to each of the above media and placed for 30 minutes. Then, the embryos were transferred to Y- medium again and placed for 30 minutes.

Then, each embryo was frozen.

Then, the embryos were then thawed and placed in M2 medium for 10 minutes.

Then, each embryo was evenly divided and placed on the medium for 1 hour.

The embryos were observed by returning to the Y- medium again. This observation was performed immediately after thawing, 3 hours later, and 24 hours later.

The results of these manipulations and processes are shown in Table 9 below.

**[Table 9]**

| COMPARING NUMBER OF EMBRYOS AFTER THAWING | | | | | | | |
|---|---|---|---|---|---|---|---|
| CULTURE MEDIUM USED | NUMBER OF EMBRYOS USED | IMMEDIATELY AFTER THAWING | RECOVERY RATE | ABOUT 3 HOURS LATER | RECOVERY RATE | ABOUT 24 HOURS LATER | RECOVERY RATE |
| Y-mWM (Control) | 19 | 1 | 5% | 10 | 52% | 7 | 36% |
| Y+mWM | 19 | 7 | 36% | 18 | 94% | 17 | 89% |
| CB+mWM(5 *µ* M) | 19 | 5 | 26% | 18 | 94% | 15 | 78% |
| CB+mWM(10 *µ* M) | 19 | 2 | 10% | 10 | 52% | 12 | 63% |

As a result, survival and recovery of many embryos in CB+ medium could be confirmed at the similar levels of those in Y+ medium as in the result of using and comparing CB+ medium with Y+ medium.

Specifically, as the result of comparison by using Y- medium (control), Y+ medium, and CB+ medium, it was confirmed that the medium by using Y-27632 had the highest survival and recovery of embryos. The next best result was CB+ medium (CB concentration is 5 µM). The data observed 3 hours after CB+ (CB concentration is 5 µM) showed good results as similar to Y+ medium. In comparison with CB+ concentration in the medium, the recovery rate of 5 µM was 15% higher than that of 10 µM at the stage after 24 hours.

The control Y-medium had the lowest recovery rate in the verification.

In addition, it is needless to say that the configuration and operation of the above-described embodiment are examples and can be appropriately modified and executed without departing from the aim of the present invention.

### [Example 2]

Next, as Example 2 of the present invention, the results of treatment examples in which the in-vitro-culture from other strains or damaged by other types of manipulations are temporarily treated are described.

For each treatment example, the materials and methods are the same as in Example 1 as described above.

The inbred mouse B6J, the combined immunodeficiency mouse NOD-scid, and the inbred rat Wistar-Imamichi according to Example 2 are all provided from CLEA Japan, Inc.

### (Treatment example 11)

By using an inbred mouse (B6J in B6 strain) different from the inbred mouse of the treatment example 2 according to the first embodiment, knock-in is performed by the same two-step method as the above-mentioned treatment example 2, and further individual preparation is performed.

The results of individual production by this 2STEP method are shown in Table 10 below.

**[Table 10]**

| GENERATION OF FLOX INBRED MICE (INDIVIDUALS) BY TWO-STEP METHOD | | | | |
|---|---|---|---|---|
| STRAIN NAME | GENE NAME | NUMBER OF FLOX INDIVIDUALS | **%** | PRESENCE OR ABSENCE OF Y |
| **B6J** | **floxed Mecp2 2STEP** (HIGH CONCENTRATION) | **2/51** | **3.9%** | PRESENCE |
| | **floxed Mecp2 2STEP** | **0/?** | **0%** | ABSENCE |
| **B6J** | **floxed Drb1 2STEP** | **1/39** | **2.6%** | PRESENCE |
| | | **0/?** | **0%** | ABSENCE |

Furthermore, in the same manner, the individual production is performed in an inbred mouse B6J strain by an electroporation method (1STEP method) by using a high concentration ssODN.

The result is shown in Table 11 below.

**[Table 11]**

| GENERATION OF FLOX INBRED MICE (INDIVIDUALS) BY ONE-STEP METHOD | | | | |
|---|---|---|---|---|
| STRAIN NAME | GENE NAME | NUMBER OF FLOX INDIVIDUALS | **%** | PRESENCE OR ABSENCE OF Y |
| **B6J** | **floxed MK2 1STEP** (HIGH CONCENTRATION) | **1/6** | **16.7%** | PRESENCE |
| | | **0** | - | ABSENCE |

Thus, even with the 1STEP method, Flox inbred mouse individuals could be produced.

### (Treatment example 12)

The pronuclear stage embryos of inbred rats similar to those of Treatment Example 6 according to Example 1 were knocked in by an electroporation method (1STEP method) by using a high concentration ssODN, and further individual preparations were performed.

As in the above-mentioned treatment example 6, insertion of the LoxP that was not knocked in at the normal ssODN concentration and the number of transfer pulses was performed with the same temporary treatment as in the above-mentioned Example 1. Specifically, before and after electroporation, 1 hour of the temporary treatment, 24 hours of culture in the normal medium (waiting period), and 1 hour of the temporary treatment were performed.

This made it possible to double the concentration of ssODN and the number of electroporations and to knocked in. Further, when the concentration of Cas9 was increased 5 times, Flox inbred rats were obtained by one electroporation.

The results are shown in Tables 12 and 13 below.

**[Table 12]**

| NO. | TRANSPLANTATION DATE | MEDIUM | STRAIN | NUMBER OF TRANSPLANTED EMBRYOS | FLOX (2 SITES) |
|---|---|---|---|---|---|
| **1** | **200409, 10** | **+Y** | **F344** (CLEA) | **92** | **1/9(11%)** |

**[Table 13]**

| GENERATION OF FLOX INBRED RATS (INDIVIDUALS) BY ONE-STEP METHOD | | | | |
|---|---|---|---|---|
| STRAIN NAME | GENE NAME | NUMBER OF FLOX INDIVIDUALS | % | PRESENCE OR ABSENCE OF Y |
| **F344** | **floxed p53 1STEP** (HIGH CONCENTRATION, MANY PULSES) | **1/9** | **11.1%** | PRESENCE |
| | | **0** | - | ABSENCE |

As a result, DNA fragments of the Flox sequence can be inserted into multiple sites of inbred rat early embryos at the same time, which are extremely difficult to manipulate, and individual preparation can be occurred after genome editing with a gRNA concentration at 2 times and a Cas9 protein concentration at 5 times.

In this way, it was possible to simultaneously edit the genomes of multiple sites in the early embryos of inbred rats and inbred mice, which are extremely difficult to manipulation.

### (Treatment example 13)

Individuals homozygous knock-outs with large deletions were produced by electroporation by using inbred rats.

In the Wistar-Imamichi line, which are similar to the inbred line, and the inbred WKY line, when the Klotho gene (40 kbp), of which whole gene could not normally be deleted, were knocked out by electroporation, the temporarily treatment is processed as similar to Example 1 as described above. Specifically, before and after electroporation, 1 hour of temporary treatment, 24 hours of culture in the normal medium (waiting period), and 1 hour of temporary treatment were performed.

As a result, knockout inbred rats lacking a gene as large as 40 kbp in length (large deletion) were obtained with high efficiency, and most of them were homozygous.

The results are shown in Tables 14 and 15 below.

**[Table 14]**

| NO. | TRANSFER DATE | MEDIUM | STRAIN | NUMBER OF TRANSFERRED EMBRYOS | **large deletion** |
|---|---|---|---|---|---|
| **1** | **200603,04** | **+Y** | **Wistar-Imamichi** | **164** | **29/46(63%)** |
| **2** | **200707** | **-Y** | **WKY** | **153** | **0/15(0%)** |
| **3** | **200903** | **+Y** | **WKY** | **15** | **2/3(66%)** |

**[Table 15]**

| GENERATION OF LARGE DELETION KO (40 KBP) INBRED STRAIN AND LARGE DELETION HOMOZYGOUS KO SEMI-INBRED STRAIN RATS (INDIVIDUALS) | | | | |
|---|---|---|---|---|
| STRAIN NAME | GENE NAME | NUMBER OF INDIVIDUALS | **%** | PRESENCE OR ABSENCE OF Y |
| **Wistar-Imamichi** | KLOTHO LARGE DELETION HETERO (HIGH CONCENTRATION) | **19/46** | **41.3%** | PRESENCE |
| | KLOTHO LARGE DELETION HOMO (HIGH CONCENTRATION) | **10/46** | **23.2%** | PRESENCE |
| | KLOTHO LARGE DELETION HETERO (LOW CONCENTRATION) | **3/18** | **16.6%** | PRESENCE |
| | KLOTHO LARGE DELETION HOMO (LOW CONCENTRATION) | **0/18** | **0%** | PRESENCE |
| **WKY** | KLOTHO LARGE DELETION (HIGH CONCENTRATION. MANY PULSES) | **2/3** | **66.6%** | PRESENCE |
| | KLOTHO LARGE DELETION | **0/15** | **0%** | ABSENCE |

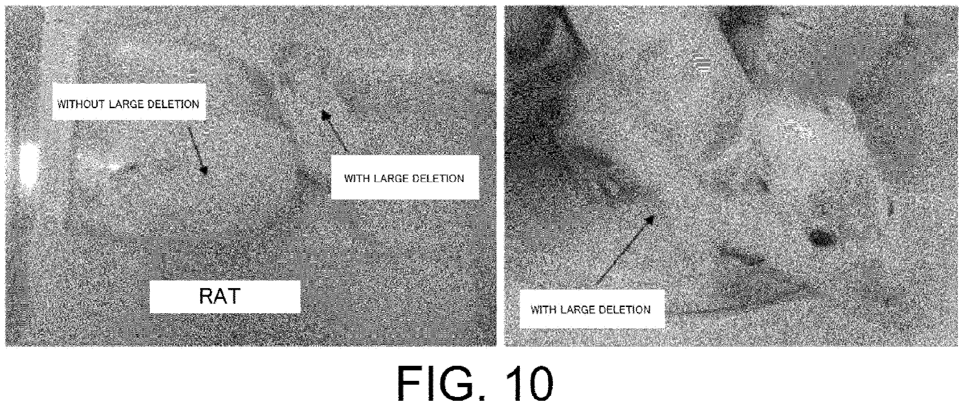

FIG. 10 shows an example of an individual that developed as a large defect knockout inbred rat. "With large deletion" incicated the large deletion knock-out inbred rat of this example, and "without large deletion" indicated the control rat.

In this way, it has become possible to produce homozygous gene-deficient individuals.

As described above, in this example, production of genome-edited individuals of inbred mice, inbred strains of heterologous mice, inbred strain rats, and inbred strain-like rats by multiple electroporations, and production of genome-edited individuals of inbred mice, inbred strains of heterologous mice, inbred strain rats, or inbred strain-like rats by introducing high concentration DNA, RNA, and protein to fertilized eggs, has become possible to produce.

### (Treatment example 14)

Then, in the same manner as in Treatment Example 7 of Example 1, in the NOD-scid strain, which is another strain of a severe combined immunodeficiency mouse, genome editing of multiple genes was performed, and individuals were produced.

Here, individuals in which both the mBBOX1 and mIL2RG genes of NOD-scid mice were knocked out were prepared by the same treatment method as the above-mentioned treatment example used for BALB/c nude.

The results are shown in Table 16 below.

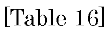

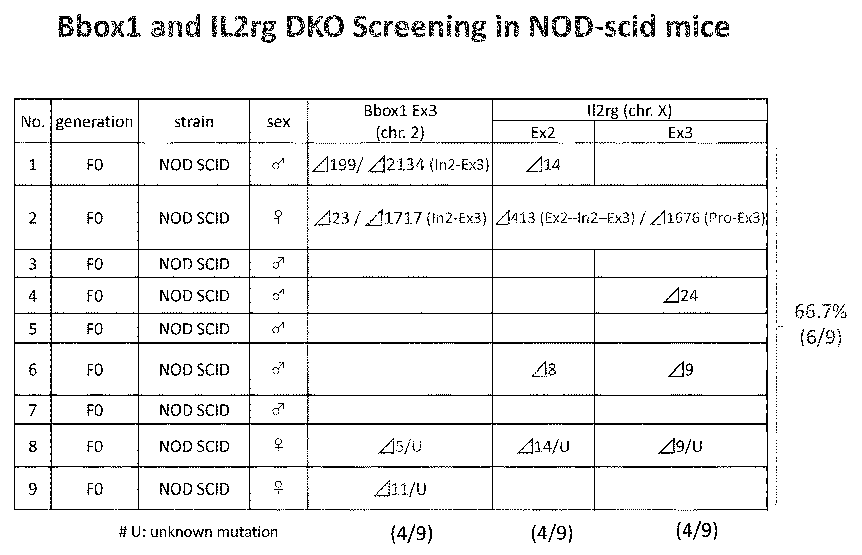

As a result, 6 out of 9 individuals (66.7%) had mutations in one of the two genes.

As described above, it has become possible to edit the genome of an immunodeficient mouse early embryo, which is extremely difficult to operate and obtain (prepare), and multiple sites of a plurality of genes, and prepare an individual.

### (Treatment example 15)

Then, in the same manner as in Treatment Example 9 according to Example 1, it was investigated whether or not the early embryos of the inbred mouse B6J strain, not the normal mice, could recover after frozen and thawed at multiple times.

The results are shown in Table 17 below.

**[Table 17]**

| INBRED MOUSE EARLY EMBRYOS CAN BE FROZEN AND THAWED MULTIPLE TIMES | | | | |
|---|---|---|---|---|
| STRAIN NAME | NUMBER OF FREEZING TIMES | NUMBER OF RECOVERIES | **%** | PRESENCE OR ABSENCE OF Y |
| **B6J** | TWICE | **24/26** | **92%** | PRESENT |
| | | **8/19** | **42%** | ABSENCE |

### (Treatment example 16)

Next, in the same manner as in Treatment Example 1 according to Example 1, a knockout by the electroporation method was also produced in an unfrozen pronuclear stage embryo (1 cell) of the inbred rat F344. The conditions at this time were that the gRNA concentration was twice the specified amount, the pulse was 14 times, and the reduction rate was 20%.

The results are shown in Table 18 below.

**[Table 18]**

| PRODUCTION OF GENOME-EDITED INDIVIDUALS BY UNFROZEN 1-CELL TAKE OF F344 (CLEA) | | | | | | |
|---|---|---|---|---|---|---|
| EMBRYO STAGE | PRESENCE/ABSENCE OF FREEZING | NUMBER OF TAKE EMBRYOS | NUMBER OF RECIPIENTS | NUMBER OF NEWBORNS (NUMBER OF NEWBORNS ANALYZED | NUMBER OF KO INDIVIDUALS **(Large deletion) (%)** | PRESENCE OR ABSENCE OF Y |
| 1 CELL | ABSENCE | 75 | 3 | 16 (13) | 7 (53.8%) | PRESENCE |
| 1 CELL | ABSENCE | 25 | 1 | 4 (4) | 0 (0%) | ABSENCE |

| | | | | | | |
|---|---|---|---|---|---|---|
| CONDITIONS: 2× gRNA CONCENTRATION, 14 PULSES, AND 20% REDUCTION RATE * KO RATE (%) IS CALCULATED BY DIVIDING THE NUMBER OF KO INDIVIDUALS BY THE NUMBER OF NEWBORNS ANALYZED. | | | | | | |

### (Treatment example 17)

Next, knock-outs by TAKE were also produced in the pronuclear stage (1 cell) and the 2-cell stage (2 cells) emplyos of the frozen and thawed inbred rat Wistar-Imamichi. The conditions at this time were that the gRNA concentration was a specified amount (1 time), the pulse was 7 times, and the reduction rate was 40%.

The results are shown in Table 19 below.

**[Table 19]**

| PRODUCTION OF GENOME-EDITED INDIVIDUALS BY FROZEN 1-2 CELL TAKE OF WISTAR-LMAMICHI | | | | | |
|---|---|---|---|---|---|
| PRESENCE OR ABSENCE OF Y | PRESENCE OR ABSENCE OF FREEZING | NUMBER OF TAKE EMBRYOS | NUMBER OF RECIPIENTS | NUMBER OF NEWBORNS | NUMBER OF KO INDIVIDUALS **(Large deletion) (%)** |
| PRESENCE | PRESENCE | 37 | 1 | 8 | 2 (25%) |
| ABSENCE | PRESENCE | 35 | 2 | NUMBER OF BIRTHS: 0 IMPLANTATION SITE: 7 DEAD FETUSES: 5 | - ( - %) |

| | | | | | |
|---|---|---|---|---|---|
| CONDITIONS: 1 × gRNA CONCENTRATION, 7 PULSES, 40% REDUCTION RATE *KO RATE (%) IS CALCULATED BY DIVIDING THE NUMBER OF KO INDIVIDUALS BY THE NUMBER OF NEWBORNS ANALYZED. | | | | | |

In this way, even in inbred mice and rats, manipulations that cause great damage, especially manipulations in which freeze-thaw is performed multiple times at a time such as the 2-cell stage in addition to pronuclear stage embryos, or the like, could be used.

### (Treatment example 18)

Next, the developmental efficiency of frozen embryo transfer by using C57BL/6J strain mouse blastocysts was examined.

Specifically, in the blastocysts of C57BL/6J mice, a freeze-thaw manipulation was performed by using the above-mentioned temporary treatment medium by using Y-27632 or the normal medium as control. Further, an average of 20 blastocysts per recipient (MCH mouse) was transplanted into the uterus. The newborn was delivered by Caesarean section (CS), and the number of implantation marks and the number of newborns were confirmed. Weaning mice were suckled by foster parents, the number of weaning mice was confirmed, and the weaning survival rate was calculated. In addition, the embryo medium was used based on the mWM medium.

The results are shown in Table 20 below.

**[Table 20]**

| DEVELOPMENTAL EFFICIENCY OF FROZEN EMBRYO TRANSFER BY USING C57BL/6J BLASTOCYSTS | | | | | | |
|---|---|---|---|---|---|---|
| **Embryo stage** | **Freeze-thawed** | **embryo, transferred** | **Implantation mark** | **Pups (CS)** | **Weaning (survival rate)** | **Y-27632** |
| blastocyst | + | 38 | 13 | 10 | 10 (100%) | + |
| blastocyst | + | 97 | 29 | 10 | 7 (70%) | - |

As a result, when frozen blastocysts were thawed and transplanted by using Y-27632, the number of offspring produced was large relative to the number of transferred blastocysts, and the weaning survival rate was also improved, as compared to when Y-27632 was not used,

Therefore, the temporary treatment medium containing Y-27632 according to the present embodiment was able to reduce the damage to the embryo and stabilize it during freezing and thawing the blastocyst.

### (Treatment example 19)

Then, the efficiency of genome editing of multiple targets was examined by using early embryos of immunodeficient mouse NOD-scid.

Electroporation (genome editing) of high-concentration RNA-protein mixed with CRISPR-Cas9 at multiple sites (4 sites in total) of a plurality of genes in early embryos of immunodeficient mice, which are extremely difficult to manipulate and prepare, was performed, transplanted into the recipient's uterus, and the offspring were obtained.

The results are shown in Table 21 below.

**[Table 21]**

| GENOME EDITING EFFICIENCY OF MULTIPLE TARGETS BY USING IMMUNODEFICIENT MOUSE NOD-scid | | | | | | |
|---|---|---|---|---|---|---|
| **Strain** | **Target genes** | **Target sites** | **Embryos, transferre d** | **Pups (KO)** | **KO %** | **Y-27632** |
| NOD-scid | Bbox1, IL2RG | 4 | 49 | 2(2) | 100 | + |
| | Bbox1, IL2RG | 4 | 47 | 7(4) | 57 | + |
| | Bbox1, IL2RG | 4 | 40 | 0(0) | - | - |

As a result, no newborn was obtained when the temporary treatment medium containing Y-27632 according to the present embodiment was not used. On the other hand, when Y-27632 was used, it withstood the stress from high-concentration RNA-protein complex and electroporation due to genome editing, and even if the number of transplanted embryos was small, newborns were obtained. Moreover, the efficiency of genome editing has been dramatically improved compared with the conventional method.

As described above, by using the temporary treatment medium containing Y-27632 according to the present embodiment, (1) the early embryos of immunodeficient mice having a high degree of difficulty in manipulation can be stabilized, and newborns can be efficiently obtained. (2) High-concentration nucleic acid-protein solution and electroporation stress can be relieved. (3) The repair efficiency of double-strand breaks (DSB) by genome editing can be increased, and the efficiency of genome editing can be improved.

Nude mice, disease model inbred rats and mice generally have low fertility. There are few fertilized eggs that can be collected, and it is difficult to manipulate the fertilized eggs in vitro. The same applies to rare varieties and endangered species. By each treatment by using the temporary treatment medium according to the present embodiment, it is possible to utilize the reproduction and reproductive engineering by using such a few rare, valuable, and fragile early embryos.

### (Treatment example 20)

Then, the passage efficiency of mice prepared by using the temporary treatment medium according to the present embodiment was examined.

The double knock-out mice of IL2RG gene and the Bbox1 gene by using the NOD-scid mice in the above-mentioned treatment example 14 were mated with each other, and it was examined whether or not the newborn mouse was affected by Y-27632.

The results are shown in Table 22 below.

**[Table 22]**

| PASSAGE EFFICIENCY OF MICE BY USING Y-27632 | | | | | |
|---|---|---|---|---|---|
| **Strain** | **generatio n** | **Pregnancy** | **male pups** | **female pups** | **Total (ave. litter size)** |
| NOG-Bbox1 (NOD-scid: Bbox1, IL2RG) | F1 | 3 | 10 | 13 | 23 (7.7/preg.) |
| | F2 | 8 | 29 | 25 | 54 (6.8/preg.) |

As a result, an average of 6 to 8 pups were born from one pair in both the first generation (F1) and the second generation (F2). This is about the same as the offspring rate of the base NOD-scid mice. In addition, there was no abnormality in the phenotype of the offspring mice.

Thus, the use of Y-27632 according to the present embodiment did not affect mouse passage and phenotype.

Here, the commercially available "NOG" strain mouse is a highly severe combined immunodeficiency mouse in which the IL2RG gene is disrupted in NOD-scid mice. Since this commercially available NOG mouse is not licensed to reproduce, it is not possible to freely produce a mouse in combination with a knockout mouse of another gene. Even in such a case, by directly producing a double knockout containing IL2RG, it can be easily used for research, and the like.

### [Industrial applicability]

According to the present invention, it is possible to provide a temporary treatment medium for reducing damage caused by manipulation of an in-vitro-culture, and it is industrially available.

## Claims

1. A temporary treatment medium for reducing manipulation damage to in-vitro-culture including any or any combination of a pluripotent stem cell, a germ cell, a fertilized egg, and an embryo, comprising:
an intracellular skeleton regulator and/or an apoptosis inhibitor.

2. The temporary treatment medium according to claim 1, wherein
the intracellular skeleton regulator and/or the apoptosis inhibitor is a Rho kinase inhibitor.

3. The temporary treatment medium according to claim 2, wherein
the Rho kinase inhibitor includes a ROCK inhibitor.

4. The temporary treatment medium according to claim 3, wherein
the ROCK inhibitor isY-27632, and
the concentration ofY-27632 is 0.1 µM to 20 µM.

5. The temporary treatment medium according to claim 1, wherein
the intracellular skeleton regulator is cytochalasin B.

6. The temporary treatment medium according to claim 5, wherein
the concentration of cytochalasin B is 0.1 µM to 15 µM.

7. The temporary treatment medium according to any one of claims 1 to 6, wherein
the in-vitro-culture is originated from Primates, Rodentia, Lagomorpha, Cetartiodactyla, Perissodactyla, or Carnivora.

8. A treatment kit including the temporary treatment medium according to any one of claims 1 to 7.

9. An embryogenesis arrest inhibitor including:
an anti-apoptotic agent, which reduces damage caused by manipulation of an in-vitro-culture and suppresses embryogenesis arrest.

10. The embryogenesis arrest inhibitor according to claim 9, wherein
the in-vitro-culture includes one or any combination of germ cells, fertilized eggs, and embryos.

11. The embryogenesis arrest inhibitor according to claim 9 or 10, wherein
the embryogenesis arrest inhibitor is for a temporary treatment medium.

12. The embryogenesis arrest inhibitor according to any one of claims 9 to 11, wherein
the apoptosis inhibitor is a Rho kinase inhibitor.

13. The embryogenesis arrest inhibitor according to claim 12, wherein
the Rho kinase inhibitor includes a ROCK inhibitor.

14. The embryogenesis arrest inhibitor according to claim 13, wherein
the ROCK inhibitor is Y-27632.

15. An embryogenesis arrest inhibitory method that reduces damage caused by manipulation of an in-vitro-culture including any or any combination of a pluripotent stem cell, a germ cell, a fertilized egg, and an embryo, and suppresses embryogenesis arrest, comprising the step of:
treating with a temporary treatment medium including an intracellular skeleton regulator and/or an apoptosis inhibitor for a specific period of time before and/or after a damaging manipulation.

16. The embryogenesis arrest inhibitory method according to claim 15, wherein
the in-vitro-culture is originated from Primates, Rodentia, Lagomorpha, Cetartiodactyla, Perissodactyla, or Carnivora.

17. The embryogenesis arrest inhibitory method according to claim 15 or 16, wherein
the specific period is within one hour when by using an animal species, a strain, and/or a frozen egg that is sensitive to the manipulation.

18. The embryogenesis arrest inhibitory method according to claim 17, wherein
the in-vitro-culture is derived from a strain of mammal that is sensitive to the manipulation.

19. The embryogenesis arrest inhibitory method according to claim 18, wherein
the manipulation is a multiple electroporation method, and
the mammal is a hybrid, a neighboring strain, a disease model, or heterogenous.

20. A developmental engineering product production method comprising the step of:
preparing a developmental engineering product including one or any combination of an individual, an organ, a tissue, and a cell from the in-vitro-culture treated by the embryogenesis arrest inhibitory method according to any one of claims 15 to 19.

21. A transplantation method comprising the step of:
transplanting the in-vitro-culture treated by the embryogenesis arrest inhibitory method according to any one of claims 15 to 19 and/or
the developmental engineering product produced by the developmental engineering product production method according to claim 20.

22. A therapeutic method for mammal comprising the step of:
transplanting the in-vitro-culture treated by the embryogenesis arrest inhibitory method according to any one of claims 15 to 19 and/or
the developmental engineering product produced by the method for producing developmental engineering product according to claim 20.

23. A developmental engineering product, wherein
being produced by the method for producing developmental engineering product according to claim 20.
